# EUROPEAN PATENT SPECIFICATION

(11) **EP 1 828 143 B1**
(45) Date of publication and mention of the grant of the patent: **20.03.2013**
(21) Application number: 05823957.5
(22) Date of filing: 15.12.2005
(51) Int. Cl.: C07D 235/02, C07D 403/04, C07D 401/04, A61K 31/4164, A61K 31/4439

(54) **2-(PHENYL OR HETEROCYCLIC)-1H-PHENANTRHO[9,10-D]IMIDAZOLES AS MPGES-1 INHIBITORS**
2-(PHENYL- ODER HETEROCYCLYL)-1H-PHENANTRO[9,10,D]IMIDAZOLE ALS MPGES-1-INHIBITOREN
1H-PHENANTHRO[9,10-D]IMIDAZOLES SUBSTITUES EN 2 PAR UN PHENYLE OU UN HETEROCYCLE EN TANT QU'INHIBITEURS DE MPGES-1

(30) Priority: 17.12.2004 US 637180 P; 23.11.2005 US 739338 P
(43) Date of publication of application: 05.09.2007
(73) Proprietor: Merck Frosst Canada Ltd., Kirkland, Québec H9H 3L1 (CA)
(72) Inventor: CHAU, Anh, Kirkland, Québec H9H 3L1 (CA); COTE, Bernard, Kirkland, Québec H9H 3L1 (CA); DUCHARME, Yves, Kirkland, Québec H9H 3L1 (CA); FRENETTE, Richard, Kirkland, Québec H9H 3L1 (CA); FRIESEN, Richard, Kirkland, Québec H9H 3L1 (CA); GAGNON, Marc, Kirkland, Québec H9H 3L1 (CA); GIROUX, Andre, Kirkland, Québec H9H 3L1 (CA); MARTINS, Evelyn, Kirkland, Québec H9H 3L1 (CA); YU, Hongping, Kirkland, Québec H9H 3L1 (CA); WU, Tom, Kirkland, Québec H9H 3L1 (CA)
(74) Representative: Buchan, Gavin MacNicol
(86) International application number: PCT/CA2005/001921
(87) International publication number: WO 2006/063466

(56) References cited:
- CA-A1- 2 223 551
- CA-A1- 2 261 426
- CA-A1- 2 426 457

## Description

### BACKGROUND OF THE INVENTION

Modulation of prostaglandin metabolism is at the center of current anti-inflammatory therapies. NSAIDs and COX-2 inhibitors block the activity of cyclooxygenases and their ability to convert arachidonic acid (AA) into prostaglandin (PG) H2. PGH2 can be subsequently metabolized by terminal prostaglandin synthases to the corresponding biologically active PGs, namely, PGI2, thromboxane (Tx) A2, PGD2, PGF2α, and PGE2. A combination of pharmacological, genetic, and neutralizing antibody approaches demonstrates the importance of PGE2 in inflammation. In many respects, disruption of PGE2-dependent signalling in animal models of inflammation can be as effective as treatment with NSAIDs or COX-2 inhibitors. The conversion of PGH2 to PGE2 by prostaglandin E synthases (PGES) may therefore represent a pivotal step in the propagation of inflammatory stimuli.

Microsomal prostaglandin E synthase-1 (mPGES-1) is an inducible PGES after exposure to pro-inflammatory stimuli. mPGES-1 is induced in the periphery and in the CNS by inflammation and represents therefore a novel target for acute and chronic inflammatory disorders. The rationale for the development of specific mPGES-1 inhibitors revolves around the hypothesis that the therapeutic utility of NSAIDs and Cox-2 inhibitors would be largely due to inhibition of pro-inflammatory PGE2 while the side effect profile would be largely due to inhibition of other prostaglandins.

The present invention is directed to novel compounds that are selective inhibitors of the microsomal prostaglandin E synthase-1 enzyme and would therefore be useful for the treatment of pain and inflammation in a variety of diseases or conditions, such as osteoarthritis, rheumatoid arthritis and acute or chronic pain. Furthermore, by selectively inhibiting the pro-inflammatory PGE2, it is believed the compounds of the invention would have a reduced potential for side effects associated with the inhibition of other prostaglandins by conventional non-steoidal anti-inflammatory drugs, such as gastrointestinal and renal toxicity.

### SUMMARY OF THE INVENTION

The invention encompasses novel compounds of Formula I 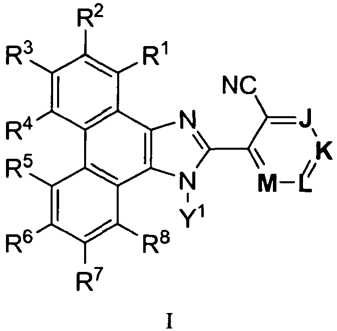 or pharmaceutically acceptable salts thereof. These compounds are inhibitors of the microsomal prostaglandin E synthase-1 (mPGES-1) enzyme and are therefore useful to treat pain and/or inflammation from a variety of diseases or conditions, such as osteoarthritis, rheumatoid arthritis and acute or chronic pain. Methods of treating diseases or conditions mediated by the mPGES-1 enzyme and pharmaceutical compositions are also encompassed.

### DETAILED DESCRIPTION OF THE INVENTION

The invention encompasses a genus of compounds represented by Formula I 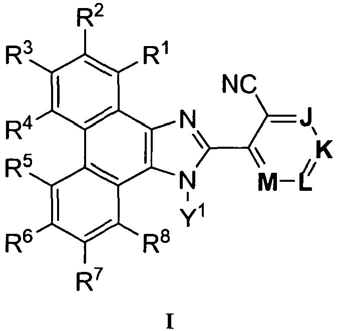 or a pharmaceutically acceptable salt of said compound, wherein:
Y¹ is H or is selected from the group consisting of: (1) C₁₋₆alkyl; (2) PO₄-C₁₋₄alkyl-; (3) C₁₋₄alkyl-C(O)-O-CH₂-, wherein the C₁₋₄alkyl portion is optionally substituted with R³³-O-C(O)-; and (4) C₁-₄alkyl-O-C(O)-;
R33 is selected from the group consisting of: (1) H; (2) C₁₋₄alkyl, (3) C₃₋₆cycloalkyl; (4) phenyl; (5) benzyl; and (6) pyridyl; said C₁₋₄alkyl, C₃₋₆cycloalkyl, phenyl, benzyl and pyridyl may each be optionally substituted with 1 to 3 substituents independently selected from the group consisting of: OH, F, Cl, Br and I;
**J** is selected from the group consisting of-C(X²)- and -N-,
**K** is selected from the group consisting of-C(X³)- and -N-,
**L** is selected from the group consisting of-C(X⁴)- and -N-, and
**M** is selected from the group consisting of-C(X⁵)- and -N-,
with the proviso that at least one of **J**, **K**, **L** or **M** is other than -N-;
X2, X³, X4 and x⁵ are independently selected from the group consisting of: (1) H; (2) -CN; (3) F; (4) Cl; (5) Br; (6) I; (7) -OH; (8) -N₃; (9) C₁₋₆alkyl, C₂₋₆alkenyl or C₂₋₆alkynyl, wherein one or more of the hydrogen atoms attached to said C₁₋₆alkyl, C₂₋₆alkenyl or C₂₋₆alkynyl may be replaced with a flouro atom, and said C₁₋₆alkyl, C₂₋₆alkenyl or C₂₋₆alkynyl may be optionally substituted with a hydroxy group; (10) C₁₋₄alkoxy; (11) NR⁹R¹⁰-C(O)-C₁₋₄alkyl-O-; (12) C₁₋₄alkyl-S(O)ₖ-; (13) NO₂; (14) C₃-₆cycloalkyl, (15) C₃₋₆cycloalkoxy; (16) phenyl, (17) carboxy; and (18) C₁₋₄alkyl-O-C(O)-;
R¹, R2, R3, R4, R5, R⁶, R⁷ and R8 are independently selected from the group consisting of: (1) H; (2) F; (3) Cl; (4) Br; (5) I; (6) -CN; (7) C₁₋₆alkyl or C₂₋₆alkenyl, wherein one or more of the hydrogen atoms attached to said C₁₋₆alkyl or C₂₋₆alkenyl may be replaced with a fluoro atom, and wherein said C₁₋₆alkyl or C₂₋₆alkenyl may be optionally substituted with one to three substituents independently selected from the group consisting of: -OH, methoxy, R¹¹-O-C(O)-, cyclopropyl, pyridyl and phenyl; (8) C₃₋₆cycloalkyl; (9) R¹²-O-; (10) R¹³-S(O)ₖ-, (11) R¹⁴-S(O)ₖ-N(R¹⁵)-; (12) R¹⁶-C(O)-; (13) R¹⁷-N(R¹⁸)-; (14) R¹⁹-N(R²⁰)-C(O)-; (15) R²¹-N(R²²)-S(O)ₖ-; (16) R²³-C(O)-N(R²⁴)-; (17) **Z**-C≡C;
(18) -(CH₃)C=N-OH or-(CH₃)C=N-OCH₃; and (19) phenyl, naphthyl, pyridyl, pyradazinyl, pyrimidinyl, pyrazinyl, pyrrolyl, pyrazolyl, imidazolyl, triazolyl, tetrazolyl, oxazolyl, isoxazolyl, oxadiazolyl, thienyl or furyl, each optionally substituted with a substituent independently selected from the group consisting of: F, Cl, Br, I, C₁₋₄alkyl, phenyl, methylsulfonyl, methylsulfonylamino, R²⁵-O-C(O)-and R²⁶-N(R²⁷)-, said C₁₋₄alkyl optionally substituted with 1 to 3 groups independently selected from halo and hydroxy;
each **Z** is independently selected from the group consisting of: (1) H; (2) C₁₋₆alkyl, wherein one or more of the hydrogen atoms attached to said C₁₋₆alkyl may be replaced with a flouro atom, and wherein
C ₁₋₆alkyl is optionally substituted with one to three substituents independently selected from: hydroxy, methoxy, cyclopropyl, phenyl, pyridyl, pyrrolyl, R²⁸-N(R²⁹)- and R³⁰-O-C(O)-; (3) -(CH₃)C=N-OH or -(CH₃)C=N-OCH₃; (4) R³¹-C(O)-; (5) phenyl; (6) pyridyl or the N-oxide thereof; (7) C₃₋₆cycloalkyl, optionally substituted with hydroxy; (8) tetrahydropyranyl, optionally substituted with hydroxy; and (9) a five-membered aromatic heterocycle containing 1 to 3 atoms independently selected from O, N or S and optionally substituted with methyl;
each R9, R¹⁰, R¹⁵, R24 and R32 is independently selected from the group consisting of: (1) H; and (2)C₁₋₄alkyl;
each R¹¹, R¹², R¹³, R¹⁴, R¹⁶, R²³, R²⁵, R³⁰ and R³¹ is independently selected from the group consisting of: (1) H; (2) C₁₋₄alkyl, (3) C₃₋₆cycloalkyl; (4) phenyl, (5) benzyl; and (6) pyridyl; said C₁-₄alkyl, C₃₋₆cycloalkyl, phenyl, benzyl and pyridyl may each be optionally substituted with 1 to 3 substituents independently selected from the group consisting of: OH, F, Cl, Br and I;
each R¹⁷, R¹⁸, R¹⁹, R²⁰, R²¹, R²², R²⁶, R²⁷, R²⁸ and R29 is independently selected from the group consisting of: (1) H; (2) C₁₋₆alkyl; (3) C₁-₆alkoxy; (4) OH and (5) benzyl or 1-phenylethyl; and R¹⁷ and R¹⁸, R¹⁹ and R20, R21 and R22, R26 and R27, and R28 and R29 may be joined together with the nitrogen atom to which they are attached to form a monocyclic ring of 5 or 6 carbon atoms, optionally containing one or two atoms independently selected from -O-, -S(O)ₖ- and -N(R³²)-; and
each k is independently 0, 1 or 2.

Within this genus, the invention encompasses a sub-genus of compounds represented by Formula A 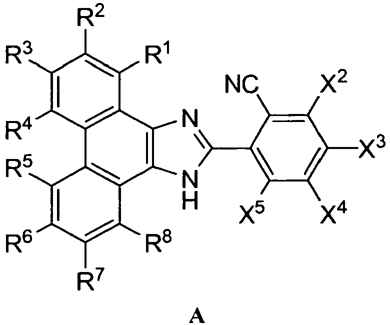 or a pharmaceutically acceptable salt of said compound.

Within this sub-genus, the invention encompasses a class of compounds of Formula **A** wherein:
X2, X³, X⁴ and X⁵ are independently selected from the group consisting of: (1) H; (2) -CN; (3) F; (4) Cl; (5) Br; and (6) I.

Also within this sub-genus, the invention encompasses a class of compounds of Formula **A** wherein X², X³ and X⁴ are H, and X⁵ is other than H. Within this class, the invention encompasses a sub-class of compounds of Formula **A** wherein X⁵ is -CN.

Also within this sub-genus, the invention encompasses a class of compounds of Formula **A** wherein at least one of R¹ or R⁸ is other than H.

Also within this sub-genus, the invention encompasses a class of compounds of Formula **A** wherein at least one of R² or R⁷ is other than H.

Also within this sub-genus, the invention encompasses a class of compounds of Formula **A** wherein at least one of R⁴ or R⁵ is other than H.

Also within this sub-genus, the invention encompasses a class of compounds of Formula **A** wherein: at least one of R³ or R⁶ is other than H; and R¹, R², R⁴, R⁵, R⁷ and R⁸ are H. Within this class, the invention encompasses a sub-class of compounds of Formula **A** wherein R³ and R⁶ are both other than H. Within this sub-class, the invention encompasses compounds of Formula **A** wherein: one of R³ or R⁶ is independently selected from the group consisting of: F, Cl, Br and I; and the other of R³ or R⁶ is **Z**-C≡C. Also within this class, the invention encompasses a sub-class of compounds of Formula **A** wherein: R3 and R6 are independently selected from the group consisting of: hydrogen, fluoro, chloro, bromo, iodo, cyano, methyl, ethyl, vinyl, cyclopropyl, -CO₂*i*-Pr, -CO₂CH₃, -SO₂CF₃, 3-pyridyl, acetyl, 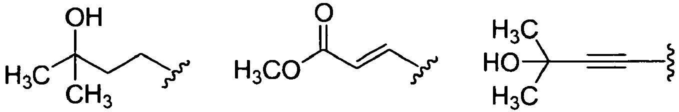 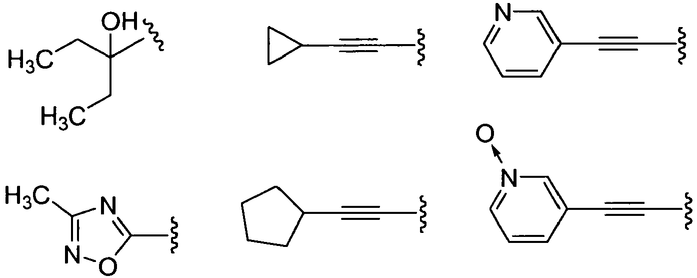 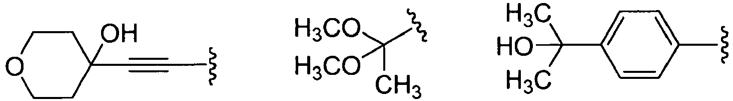 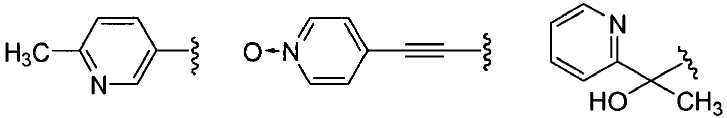 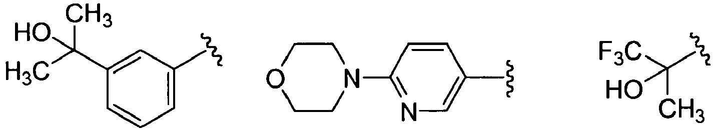 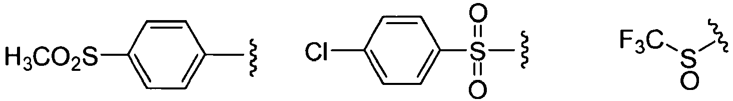 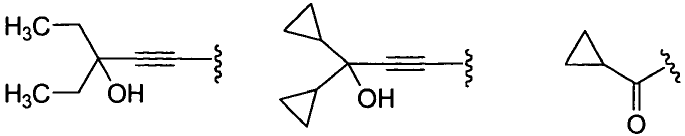 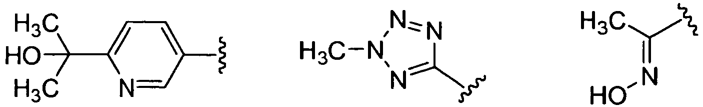 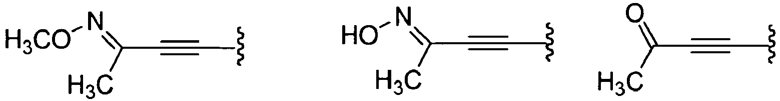 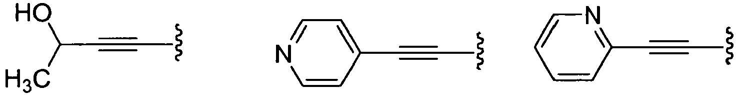 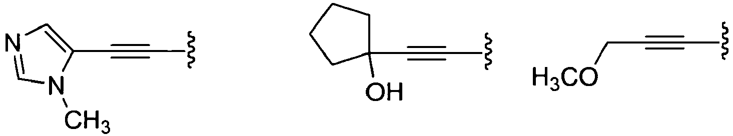 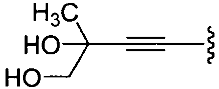 with the proviso that at least one of R3 or R⁶ is other than H.

Within the genus previously described, the invention encompasses a sub-genus of compounds of Formula **B**: 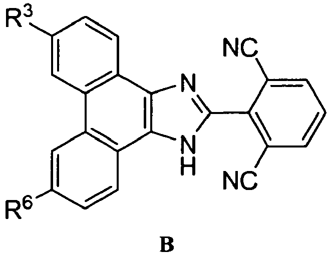 or a pharmaceutically acceptable salt of said compound. Within this sub-genus, the invention encompasses a class of compounds of Formula **B** wherein: one of R³ or R⁶ is independently selected from the group consisting of: F, Cl, Br and I; and the other of R³ or R⁶ is **Z**-C≡C.

Within the genus previously described, the invention encompasses a sub-genus of compounds of Formula I in accordance with Formula **C** 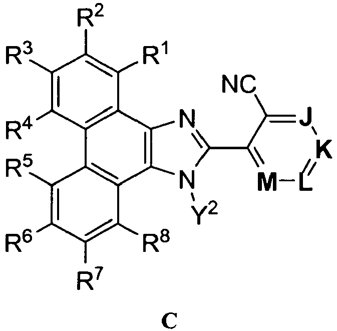 or a pharmaceutically acceptable salt thereof, wherein:
Y² is selected from the group consisting of: (1) C₁₋₆alkyl; (2) PO₄-C₁₋₄alkyl-; (3) C ₁₋₄alkyl-C(O)-O-CH₂-, wherein the C₁₋₄alkyl portion is optionally substituted with R³³-O-C(O)-; and (4) C₁₋₄alkyl-O-C(O)-; and
R³³ is selected from the group consisting of: (1) H; (2) C₁₋₄alkyl, (3) C₃₋₆cycloalkyl; (4) phenyl; (5) benzyl; and (6) pyridyl; said C₁₋₄alkyl, C₃₋₆cycloalkyl, phenyl, benzyl and pyridyl may each be optionally substituted with I to 3 substituents independently selected from the group consisting of: OH, F, Cl, Br and I.

Uses of the invention include a pharmaceutical composition comprising a compound of Formula I in combination with a pharmaceutically acceptable carrier.

The invention also encompasses a method for treating a microsomal prostaglandin E synthase-1 mediated disease or condition in a human patient in need of such treatment comprising administering to said patient a compound according to Claim 1 in an amount effective to treat the microsomal prostaglandin E synthase-1 mediated disease or condition, in particular: acute or chronic pain, osteoarthritis, rheumatoid arthritis, bursitis, ankylosing spondylitis and primary dysmenorrhea.

The following compounds exemplify the invention. These compounds were synthesized following the schemes and examples described below.

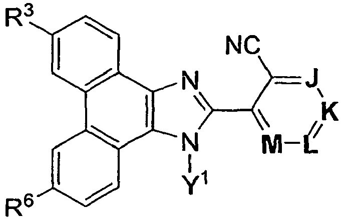

| **Ex** | **R³/R⁶** | **R⁶/R³** | **J** | **K** | **L** | **M** | **Y¹** |
|---|---|---|---|---|---|---|---|
| 1 | Cl | Br | CH | CH | CH | CF | H |
| 2 | H | H | CH | CH | CH | CH | H |
| 3 | CN | 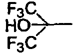 | CH | CH | CH | CF | H |
| 4 | Cl | 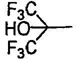 | CH | CH | CH | CF | H |
| 5 | Cl | H | CH | CH | CH | CF | H |
| 6 | CN | H | CH | CH | CH | CF | H |
| 7 | CN | 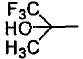 | CH | CH | CH | CF | H |
| 8 | Cl | 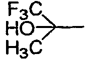 | CH | CH | CH | CF | H |
| 9 | Br | Br | CH | CH | CH | CF | H |
| 10 | H | H | CH | CH | CH | CCl | H |
| 11 | H | H | CH | CH | CH | CCN | H |
| 12 | 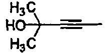 | Br | CH | CH | CH | CF | H |
| 13 | 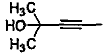 | 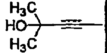 | CH | CH | CH | CF | H |
| 14 | 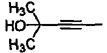 | Cl | CH | CH | CH | CF | H |
| 15 | 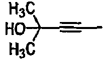 | I | CH | CH | CH | CF | H |
| 16 | H | H | CH | CH | CH | CBr | H |
| 17 | H | H | CH | CH | CH | CF | H |
| 18 | H | H | CH | N | CH | CCl | H |
| 19 | 3-pyridyl | 3-pyridyl | CH | CH | CH | CF | H |
| 20 | Cl | 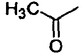 | CH | CH | CH | CF | H |
| 21 | Cl | 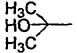 | CH | CH | CH | CF | H |
| 22 | 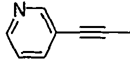 | Br | CH | CH | CH | CF | H |
| 23 | Cl | H | CH | N | CH | CCN | H |
| 24 | H | H | CH | N | CH | CCN | H |
| 25 | Cl | H | CH | CH | CH | CCN | H |
| 26 | H | H | CH | N | CH | CH | H |
| 27 | 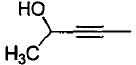 | Br | CH | CH | CH | CF | H |
| 28 | 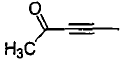 | Br | CH | CH | CH | CF | H |
| 29 | 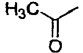 | 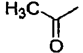 | CH | CH | CH | CF | H |
| 30 | 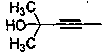 | 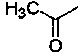 | CH | CH | CH | CF | H |
| 31 | H | H | N | CH | CH | N | H |
| 32 | H | H | N | CH | CH | CH | H |
| 33 | Br | 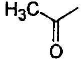 | CH | CH | CH | CF | H |
| 34 | I | I | CH | CH | CH | CF | H |
| 35 | Br | 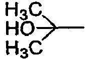 | CH | CH | CH | CF | H |
| 36 | Br | Cl | CH | CH | CH | CCN | H |
| 37 | Cl | 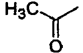 | CH | CH | CH | CBr | H |
| 38 | Cl | 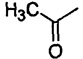 | CH | CH | CH | CCN | H |
| 39 | I | I | CH | CH | CH | CCN | H |
| 40 | 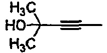 | Cl | CH | CH | CH | CCN | H |
| 41 | Cl | 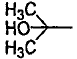 | CH | CH | CH | CCN | H |
| 42 | 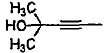 | I | CH | CH | CH | CCN | H |
| 43 | 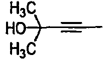 | 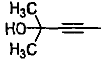 | CH | CH | CH | CCN | H |
| 44 | H | H | CH | CH | CH | CCN | CO₂Et |
| 45 | H | H | CH | CH | CH | CCN | 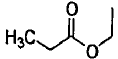 |
| 46 | 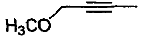 | Cl | CH | CH | CH | CCN | H |
| 47 | 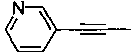 | Cl | CH | CH | CH | CCN | H |
| 48 | 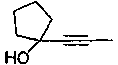 | Cl | CH | CH | CH | CCN | H |
| 49 | 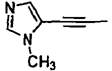 | Cl | CH | CH | CH | CCN | H |
| 50 | 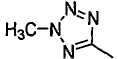 | Cl | CH | CH | CH | CCN | H |
| 51 | Cl | 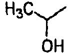 | CH | CH | CH | CCN | H |
| 52 | 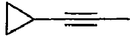 | Cl | CH | CH | CH | CCN | H |
| 53 | 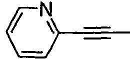 | Cl | CH | CH | CH | CCN | H |
| 54 | 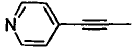 | Cl | CH | CH | CH | CCN | H |
| 55 | 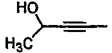 | Cl | CH | CH | CH | CCN | H |
| 56 | 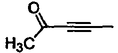 | Cl | CH | CH | CH | CCN | H |
| 57 | 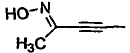 | Cl | CH | CH | CH | CCN | H |
| 58 | 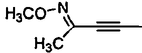 | Cl | CH | CH | CH | CCN | H |
| 59 | H | H | CH | CH | CH | CCN | 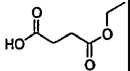 |
| 60 | H | H | CH | CH | CH | CCN | H₂PO₄CH₂ |
| 61 | 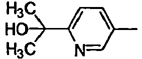 | Cl | CH | CH | CH | CCN | H |
| 62 | Cl | SO₂CH3 | CH | CH | CH | CCN | H |
| 63 | Cl | 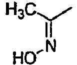 | CH | CH | CH | CCN | H |
| 64 | Br | H | CH | CH | CH | CCN | H |
| 65 | Cl | 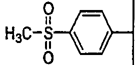 | CH | CH | CH | CCN | H |
| 66 | I | H | CH | CH | CH | CCN | H |
| 67 | CN | H | CH | CH | CH | CCN | H |
| 68 | cyclopropyl | Cl | CH | CH | CH | CCN | H |
| 69 | 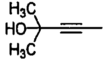 | 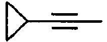 | CH | CH | CH | CCN | H |
| 70 | Cl | F | CH | CH | CH | CCN | H |
| 71 | Cl | 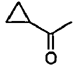 | CH | CH | CH | CCN | H |
| 72 | Cl | 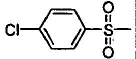 | CH | CH | CH | CCN | H |
| 73 | vinyl | H | CH | CH | CH | CCN | H |
| 74 | ethyl | H | CH | CH | CH | CCN | H |
| 75 | cyclopropyl | H | CH | CH | CH | CCN | H |
| 76 | Cl | 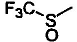 | CH | CH | CH | CBr | H |
| 77 | Cl | 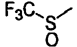 | CH | CH | CH | CCN | H |
| 78 | Cl | SO₂CF₃ | CH | CH | CH | CCN | H |
| 79 | 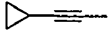 | H | CH | CH | CH | CCN | H |
| 80 | Cl | 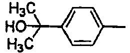 | CH | CH | CH | CCN | H |
| 81 | 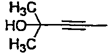 | Br | CH | CH | CH | CCN | H |
| 82 | Cl | 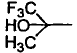 | CH | CH | CH | CCN | H |
| 83 | 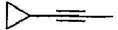 | 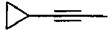 | CH | CH | CH | CCN | H |
| 84 | 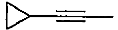 | 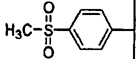 | CH | CH | CH | CCN | H |
| 85 | 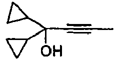 | Cl | CH | CH | CH | CCN | H |
| 86 | 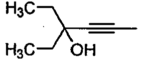 | Cl | CH | CH | CH | CCN | H |
| 87 | Br | 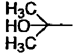 | CH | CH | CH | CCN | H |
| 88 | 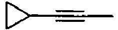 | 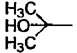 | CH | CH | CH | CCN | H |
| 89 | 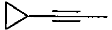 | CN | CH | CH | CH | CCN | H |
| 90 | 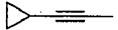 | CO₂CH₃ | CH | CH | CH | CCN | H |
| 91 | 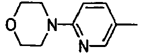 | Cl | CH | CH | CH | CCN | H |
| 92 | Cl | CN | CH | CH | CH | CCN | H |
| 93 | Cl | 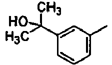 | CH | CH | CH | CCN | H |
| 94 | Br | 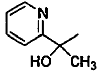 | CH | CH | CH | CCN | H |
| 95 | 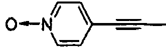 | Cl | CH | CH | CH | CCN | H |
| 96 | 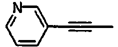 | 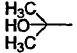 | CH | CH | CH | CCN | H |
| 97 | 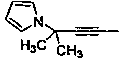 | Cl | CH | CH | CH | CCN | H |
| 98 | 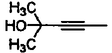 | Br | CH | CH | CH | CCl | H |
| 99 | 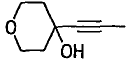 | Br | CH | CH | CH | CCl | H |
| 100 | Cl | CO₂i-Pr | CH | CH | CH | CCN | H |
| 101 | Cl | 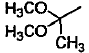 | CH | CH | CH | CF | H |
| 102 | 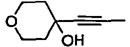 | Br | CH | CH | CH | CCN | H |
| 103 | 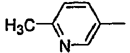 | Cl | CH | CH | CH | CCN | H |
| 104 | Br | 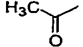 | CH | CH | CH | CCN | H |
| 105 | 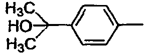 | Cl | CH | CH | CH | CCl | H |
| 106 | Br | 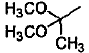 | CH | CH | CH | CCN | H |
| 107 | 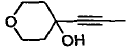 | Cl | CH | CH | CH | CCl | H |
| 108 | 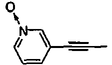 | Cl | CH | CH | CH | CCN | H |
| 109 | 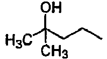 | Br | CH | CH | CH | CCN | H |
| 110 | 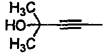 | Cl | CH | CH | CH | CCl | H |
| 111 | 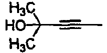 | 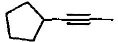 | CH | CH | CH | CCN | H |
| 112 | 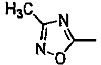 | Br | CH | CH | CH | CCN | H |
| 113 | 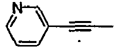 | 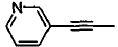 | CH | CH | CH | CCN | H |
| 114 | Et | 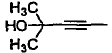 | CH | CH | CH | CCN | H |
| 115 | 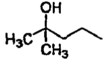 | 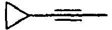 | CH | CH | CH | CCN | H |
| 116 | Br | 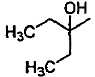 | CH | CH | CH | CCN | H |
| 117 | 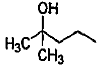 | Cl | CH | CH | CH | CCN | H |
| 118 | Br | CH3 | CH | CH | CH | CCN | H |
| 119 | 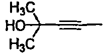 | CH3 | CH | CH | CH | CCN | H |
| 120 | 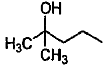 | CH3 | CH | CH | CH | CCN | H |
| 121 | 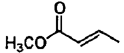 | Cl | CH | CH | CH | CCN | H |
| 122 | 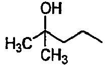 | H | CH | CH | CH | CCN | H |
| 123 | 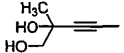 | Cl | CH | CH | CH | CCN | H |

The invention includes, as appropriate, pharmaceutically acceptable salts of any of the aforementioned compounds. For purposes of this specification, the heading "R³/R6" means that the substituent indicated in that column is substituted at the position represented by either R³ or R⁶. In the adjacent column, the heading "R⁶/R³ means the indicated substituent is substituted at the position R³ or R⁶ not substituted in the previous column. By way of example, Example 6 represents R³=CN and R⁶=H or R3=H and R⁶=CN, representing both tautomers.

The term "halogen" or "halo" includes F, Cl, Br, and I.

The term "alkyl" means linear or branched structures and combinations thereof, having the indicated number of carbon atoms. Thus, for example, C₁₋₆alkyl includes methyl, ethyl, propyl, 2-propyl, s- and t-butyl, butyl, pentyl, hexyl and 1,1-dimethylethyl.

The term "alkenyl" means linear or branched structures and combinations thereof, of the indicated number of carbon atoms, having at least one carbon-to-carbon double bond, wherein hydrogen may be replaced by an additional carbon-to-carbon double bond. C₂₋₆alkenyl, for example, includes ethenyl, propenyl, 1-methylethenyl, butenyl and the like.

The term "alkynyl" means linear or branched structures and combinations thereof, of the indicated number of carbon atoms, having at least one carbon-to-carbon triple bond. C₃₋₆alkynyl, for example, includes , propenyl, 1-methylethenyl, butenyl and the like.

The term "alkoxy" means alkoxy groups of a straight, branched or cyclic configuration having the indicated number of carbon atoms. C₁₋₆alkoxy, for example, includes methoxy, ethoxy, propoxy, isopropoxy, and the like.

The term "cycloalkyl" means mono-, bi- or tri-cyclic structures, optionally combined with linear or branched structures, having the indicated number of carbon atoms. Examples of cycloalkyl groups include cyclopropyl, cyclopentyl, cycloheptyl, adamantyl, cyclododecylmethyl, 2-ethyl-1-bicyclo[4.4.0]decyl, cyclobutylmethyl cyclopropylmethyl and the like.

Compounds described herein may contain an asymmetric center and may thus exist as enantiomers. Where the compounds according to the invention possess two or more asymmetric centers, they may additionally exist as diastereomers. The present invention includes all such possible stereoisomers as substantially pure resolved enantiomers, racemic mixtures thereof, as well as mixtures of diastereomers. The above Formula I is shown without a definitive stereochemistry at certain positions. The present invention includes all stereoisomers of Formula I and pharmaceutically acceptable salts thereof. Diastereoisomeric pairs of enantiomers may be separated by, for example, fractional crystallization from a suitable solvent, and the pair of enantiomers thus obtained may be separated into individual stereoisomers by conventional means, for example by the use of an optically active acid or base as a resolving agent or on a chiral HPLC column. Further, any enantiomer or diastereomer of a compound of the general Formula I may be obtained by stereospecific synthesis using optically pure starting materials or reagents of known configuration.

Some of the compounds described herein contain olefinic double bonds, and unless specified otherwise, are meant to include both E and Z geometric isomers.

Some of the compounds described herein may exist with different points of attachment of hydrogen, referred to as tautomers. The compound of Formula I exists in the following tautomeric forms: 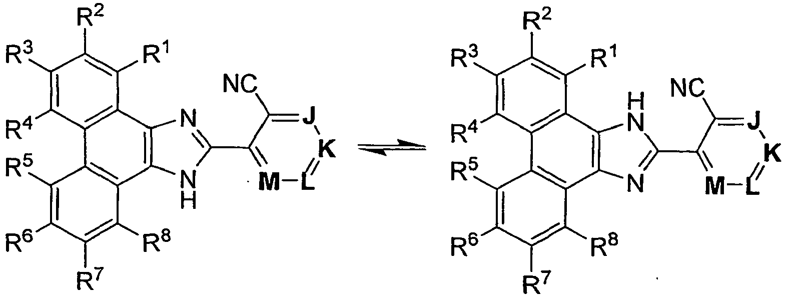 The individual tautomers as well as mixture thereof are encompassed within Formula I.

The present invention includes within its scope prodrugs of the compounds of this invention. In general, such prodrugs will be functional derivatives of the compounds of this invention which are readily convertible *in vivo* into the required compound. Thus, in the methods of treatment of the present invention, the term "administering" shall encompass the treatment of the various conditions described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs," ed. H. Bundgaard, Elsevier, 1985. Metabolites of these compounds include active species produced upon introduction of compounds of this invention into the biological milieu. Exemplifying prodrugs of the invention are compounds of Formula C.

The term "treating a microsomal prostaglandin E synthase-1 mediated disease or condition" means treating or preventing any disease or condition that is advantageously treated or prevented by inhibiting the microsomal prostaglandin E synthase-1 (mPGES-1) enzyme. The term includes the relief of pain, fever and inflammation of a variety of conditions including rheumatic fever, symptoms associated with influenza or other viral infections, common cold, low back and neck pain, dysmenorrhea, headache, migraine (acute and prophylactic treatment), toothache, sprains and strains, myositis, neuralgia, synovitis, arthritis, including rheumatoid arthritis, degenerative joint diseases (osteoarthritis), gout and ankylosing spondylitis, acute, subacute and chronic musculoskeletal pain syndromes such as bursitis, burns, injuries, and pain following surgical and dental procedures as well as the preemptive treatment of surgical pain. In addition, the term includes the inhibition cellular neoplastic transformations and metastic tumor growth and hence the treatment of cancer. The term also includes the treatment of endometriosis and Parkinson's disease as well as the treatment of mPGES-1 mediated proliferative disorders such as may occur in diabetic retinopathy and tumor angiogenesis. The term "treating" encompasses not only treating a patient to relieve the patient of the signs and symptoms of the disease or condition but also prophylactically treating an asymptomatic patient to prevent the onset or progression of the disease or condition.

The term "amounts that are effective to treat" is intended to mean that amount of a drug or pharmaceutical agent that will elicit the biological or medical response of a tissue, a system, animal or human that is being sought by a researcher, veterinarian, medical doctor or other clinician. The term also encompasses the amount of a pharmaceutical drug that will prevent or reduce the risk of occurrence of the biological or medical event that is sought to be prevented in a tissue, a system, animal or human by a researcher, veterinarian, medical doctor or other clinician. Suitable dosage levels of the compound of Formula I used in the present invention are described below. The compound may be administered on a regimen of once or twice per day.

The pharmaceutical compositions of the present invention comprise a compound of Formula I as an active ingredient or a pharmaceutically acceptable salt, thereof, and may also contain a pharmaceutically acceptable carrier and optionally other therapeutic ingredients. The term "pharmaceutically acceptable salts" include salts prepared from bases that result in non-toxic pharmaceutically acceptable salts, including inorganic bases and organic bases. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

When the compound of the present invention is basic, salts may be prepared from acids that result in pharmaceutically acceptable salts, including inorganic and organic acids. Such acids include acetic, adipic, aspartic, 1,5-naphthalenedisulfonic, benzenesulfonic, benzoic, camphorsulfonic, citric, 1,2-ethanedisulfonic, ethanesulfonic, ethylenediaminetetraacetic, fumaric, glucoheptonic, gluconic, glutamic, hydriodic, hydrobromic, hydrochloric, isethionic, lactic, maleic, malic, mandelic, methanesulfonic, mucic, 2-naphthalenesulfonic, nitric, oxalic, pamoic, pantothenic, phosphoric, pivalic, propionic, salicylic, stearic, succinic, sulfuric, tartaric, p-toluenesulfonic acid, undecanoic, 10-undecenoic, and the like.

By virtue of the mPGES-1 inhibitory activity of compounds of the present invention, the compounds of Formula I are useful for the relief of pain, fever and inflammation of a variety of conditions including rheumatic fever, symptoms associated with influenza or other viral infections, common cold, low back and neck pain, dysmenorrhea, headache, migraine (acute and prophylactice treatment), toothache, sprains and strains, myositis, neuralgia, synovitis, arthritis, including rheumatoid arthritis, degenerative joint diseases (osteoarthritis), gout and ankylosing spondylitis, acute, subacute and chronic musculoskeletal pain syndromes such as bursitis, burns, injuries, and pain following surgical and dental procedures as well as the preemptive treatment of surgical pain. In addition, such a compound may inhibit cellular neoplastic transformations and metastic tumor growth and hence can be used in the treatment of cancer. Compounds of Formula I may also be useful for the treatment or prevention of endometriosis and Parkinson's disease.

Compounds of Formula I will also inhibit prostanoid-induced smooth muscle contraction by preventing the synthesis of contractile prostanoids and hence may be of use in the treatment of dysmenorrhea, premature labor and asthma.

By virtue of their selective inhibition of the mPGES-1 enzyme, the compounds of Formula I will prove useful as an alternative to conventional non-steroidal antiinflammatory drugs (NSAID'S) particularly where such non-steroidal antiinflammatory drugs may be contra-indicated such as in patients with peptic ulcers, gastritis, regioal enteritis, ulcerative colitis, diverticulitis or with a recurrent history of gastrointestinal lesions; GI bleeding, coagulation disorders including anemia such as hypoprothrombinemia, haemophilia or other bleeding problems (including those relating to reduced or impaired platelet function); kidney disease (e.g. impaired renal function); those prior to surgery or taking anticoagulants; and those susceptible to NSAID induced asthma.

Similarly, compounds of Formula I will be useful as a partial or complete substitute for conventional NSAIDs in preparations wherein they are presently co-administered with other agents or ingredients. Thus in further aspects, the invention encompasses pharmaceutical compositions for treating mPGES-1 mediated diseases as defined above comprising a non-toxic therapeutically effective amount of the compound of Formula I as defined above and one or more ingredients such as another pain reliever including acetominophen or phenacetin; opioid analgesics, such as codeine, fentanyl, hydromorphone, levorphanol, meperidine, methadone, morphine, oxycodone, oxymorphine, propoxyphene, buprenorphine, butorphanol, dezocine, nalbuphine and pentazocine; a potentiator including caffeine; an H2-antagonist; aluminum or magnesium hydroxide; simethicone; a decongestant including phenylephrine, phenylpropanolamine, pseudophedrine, oxymetazoline, ephinephrine, naphazoline, xylometazoline, propylhexedrine, or levo-desoxyephedrine; an antitussive including codeine, hydrocodone, caramiphen, carbetapentane, or dextramethorphan; a diuretic; a sedating or non-sedating antihistamine; and a proton pump inhibitor, such as omeprazole. For the treatment or prevention of migraine, the invention also encompasses co-administration with a 5-HT agonist such as rizatriptan, sumatriptan, zolmitriptan and naratriptan. In addition the invention encompasses a method of treating mPGES-1 mediated diseases comprising: administration to a patient in need of such treatment a non-toxic therapeutically effect amount of the compound of Formula I, optionally co-administered with one or more of such ingredients as listed immediately above.

As indicated above, pharmaceutical compositions for treating mPGES-1 mediated diseases as defined may optionally include one or more ingredients as listed above.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Patent 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethyl-cellulose, methylcellulose, hydroxypropylmethy-cellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose, saccharin or aspartame.

Liquid formulations include the use of self-emulsyfying drug delivery systems and NanoCrystal^{®} technology. Cyclodextrin inclusion complexes can also be utilized.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of Formula I may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compound of Formula I are employed. (For purposes of this application, topical application shall include mouth washes and gargles.)

Pharmaceutical compositions of the invention may also utilize absorption enhancers such as tween 80, tween 20, Vitamin E TPGS (d-alpha-tocopheryl polyethylene glycol 1000 succinate) and Gelucire^{®}.

Dosage levels of the order of from about 0.01 mg to about 140 mg/kg of body weight per day are useful in the treatment of the above-indicated conditions, or alternatively about 0.5 mg to about 7 g per patient per day. For example, inflammation may be effectively treated by the administration of from about 0.01 to 50 mg of the compound per kilogram of body weight per day, or alternatively about 0.5 mg to about 3.5 g per patient per day, preferably 2.5 mg to 1 g per patient per day.

The amount of active ingredient that may be combined with the carrier materials to produce a single dosage form will vary depending upon the host treated and the particular mode of administration. For example, a formulation intended for the oral administration of humans may contain from 0.5 mg to 5 g of active agent compounded with an appropriate and convenient amount of carrier material which may vary from about 5 to about 95 percent of the total composition. Dosage unit forms will generally contain between from about 1 mg to about 500 mg of an active ingredient, typically 25 mg, 50 mg, 100 mg, 200 mg, 300 mg, 400 mg, 500 mg, 600 mg, 800 mg, or 1000 mg. Dosage amounts of 4 mg, 8 mg, 18 mg, 20 mg, 36 mg, 40 mg, 80 mg, 160 mg, 320 mg and 640 mg may also be employed. The following table exemplifies formulations that may be employed for the present invention.

It will be understood, however, that the specific dose level for any particular patient will depend upon a variety of factors including the age, body weight, general health, sex, diet, time of administration, route of administration, rate of excretion, drug combination and the severity of the particular disease undergoing therapy.

### Methods of Synthesis

The compounds of Formula I of the present invention can be prepared according to the synthetic routes outlined in Schemes 1 and 4 below and by following the methods described therein. The imidazole of Formula **I** may be prepared in a multi-step sequence from the requisite phenanthrenequinone **i.** The phenanthrene imidazole **iii** is obtained by treating the phenanthrenequinone **i** and an appropriately substituted aldehyde **ii** with a reagent such as NH₄OAc or NH₄HCO₃ in a solvent such as acetic acid. Treatement of the imidazole **iii** with CuCN in a solvent such as DMF or DMSO produces the mono or bis-nitrile (M = CCN) **Ia.** Subsequent functional group interconversion can be done at any of the R¹ to R⁸ positions. For example, if one or more of the R¹ to R⁸ substituents equal Cl, Br or I and if M is different from CBr or CI, **Ia** could be converted to **Ib** by placing **Ia** in the presence of a monosubstituted alkynyl, a stannane, a boronic acid, a borane or a boronate under conditions that promote cross coupling reaction, such as heating in the presence of a catalyst, such as Pd(PPh₃)₄ and CuI, in the presence of a base, such as sodium carbonate or diisopropylamine, and in an suitable solvent, such as THF, DMF or DME. This last exemplified step, or any other appropriate functional group transformation, can be iteratively repeated on R¹ to R⁸.

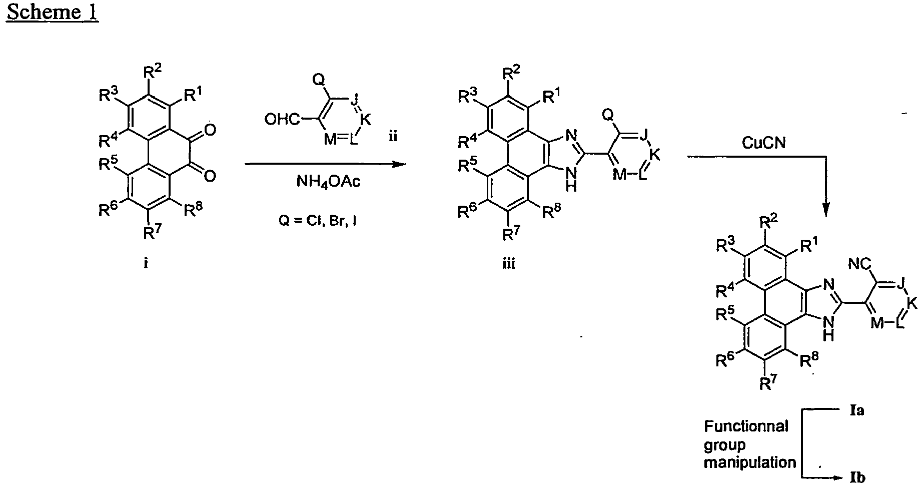

Phenanthrenequinone i can be prepared according to the sequences outlined in Scheme 2 and 3. Deprotonation of the phosphonium salt **iv** (Scheme 2) in the presence of a base, such as sodium hydride or sodium methoxide, in a solvent such as DMF followed by the addition of the aldehyde **v** produces the stylbene **vi** as a mixture of E and Z isomers. Intramolecular cyclisation of this mixture upon exposition to UV light in the presence of an oxidizing agent, such as iodine, and an acid scavenger, such as propylene oxide, in a suitable solvent such as cyclohexanne produces the phenanthrene **vii**. This phenanthrene viia can be directly oxidized with an oxidizing agent, such as CrO₃, in a suitable solvent, such as acetic acid, to provide the phenanthrenequinone **i**, or optionally, phenanthrene **viia** could be further elaborated to phenanthrene **viib** by the appropriate interconversion of any of the functional group R¹ to R⁸, such as transmetallation with an organometallic reagent, such as butyl lithium, in a suitable solvent such as THF, followed by the addition of an electrophile, such as iodine or carbon dioxide. Alternatively (Scheme 3), phenylacetic acid **viii** can be condensed with the aldehyde **ix** in the presence of a base, such as potassium carbonate, and in the presence of acetic anhydride to afford the nitro stylbene **x.** This nitro aryl **x** is then reduced with an appropriate reducing agent, such as iron or iron sulfate, in the presence of ammonium hydroxide in a suitable solvent, such as acetic acid, to produce the amine **xi.** Diazotization of this amine **xi** with sodium nitrite in the presence of aqueous hydroxide, such as sodium hydroxide, followed by acidification with an acid, such as sulfuric acid and sulfamic acid, and cyclization in the presence of a catalyst, such as copper or a ferrocene, generates the phenanthrene carboxylic acid **xii**. This phenanthrene can be oxidized and simultaneously decarboxylated using an appropriate oxidizing agent, such as chromium trioxide in suitable solvent, such as acetic acid, to afford the phenanthrenequinone **i**.

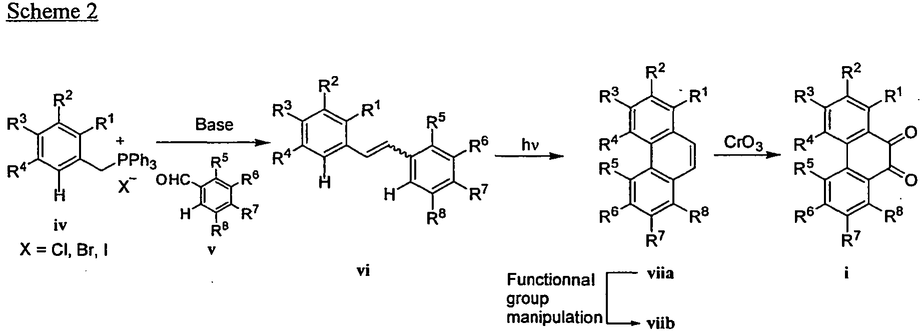

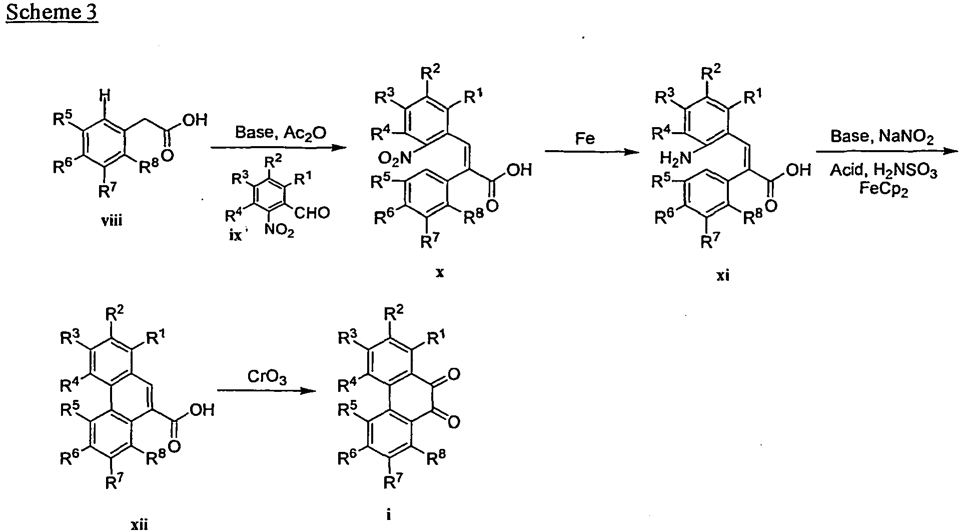

As shown in Scheme 4, protection of the halophenanthrene **xiii** with an appropriate protecting group such as 2-(trimethylsily)ethoxymethyl in the presence of a base, such as sodium hydride or diisopropylethylamine, in a suitable solvent, such as DMF or methylene chloride, affords the protected phenanthrene imidazole **xiv.** This phenanthrene imidazole **xiv** is then carbonylated with carbon monoxide in the presence of a catalyst, such as Pd(OAc)_{2,} and in the presence of a base, such as triethylamine, in a mixture of an alcoholic solvent, such as methanol and DMF, or any other suitable organic solvent. Treatment of the ester **xv** with a nucleophilic reagent such as an organolithium, organocerium or Grignard reagent in an organic solvent, such as ether, THF or methylene chloride (Grinard reagent), provides the tertiary alcohol **xvi.** Removal of the imidazole protecting group, for example by treating xvi with a mineral acid such as hydrochloric acid or in the presence of a fluoride source such as TBAF, in an organic solvent such as THF, affords the unprotected imidazole **xvii.** Treatment of this phenanthrene imidazole **xvii** with CuCN in a solvent, such as DMF or DMSO, produced the mono or bis-nitrile (M = CCN) **Id**. Subsequent functional group interconversion can be done at any of the R¹ to R⁸ positions. For example, if one or more of the R¹ to R8 substituents equal Cl, Br or I and if M is different from CBr or CI, **Id** could be converted to **Ie** by placing **Id** in the presence of a monosubstituted alkynyl, a stannane, a boronic acid, a borane or a boronate under conditions that promote cross coupling reaction, such as heating in the presence of a catalyst such as Pd(PPh₃)₄ and CuI, and in the presence of a base, such as sodium carbonate or diisopropylamine, in a suitable solvent, such as THE, DMF or DME. This last exemplified step, or any other appropriate functional group transformation, can be iteratively repeated on R¹ to R⁸.

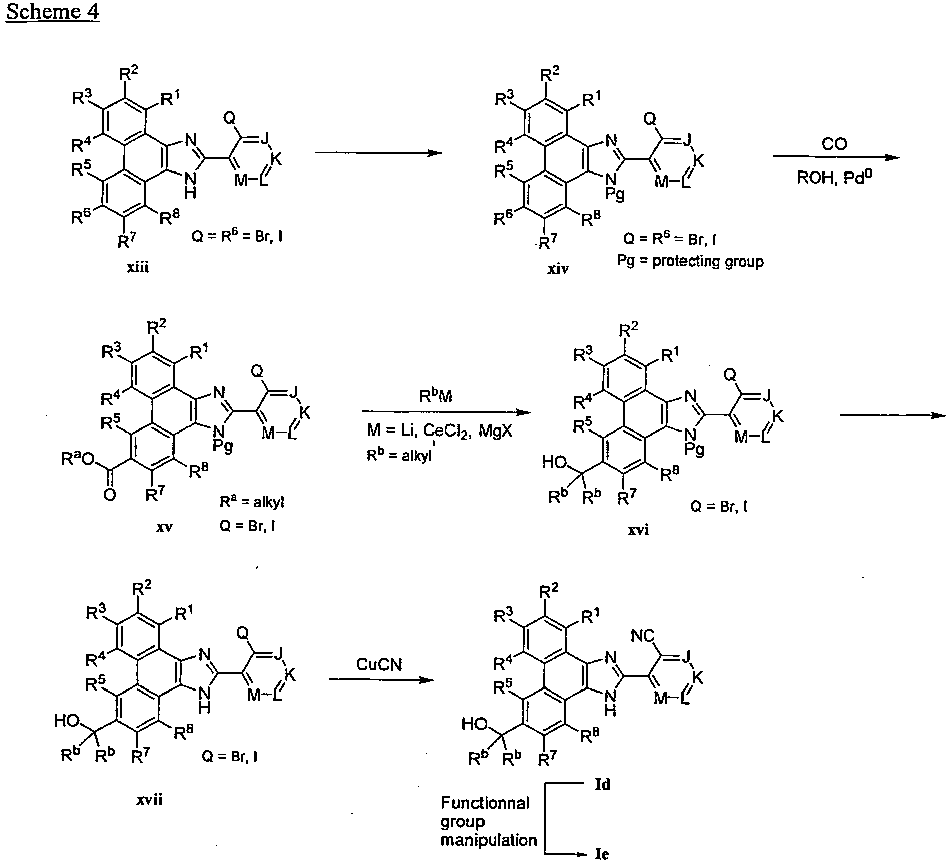

The imidazole secondary amine can be substituted as described in Scheme 5 by treating an appropriately functionalized phenanthrene imidazole I with a reagent such as an acylating agent or an alkylating agent such as methyl iodide in the presence of a base such as sodium hydride in a suitable solvent such as DMF.

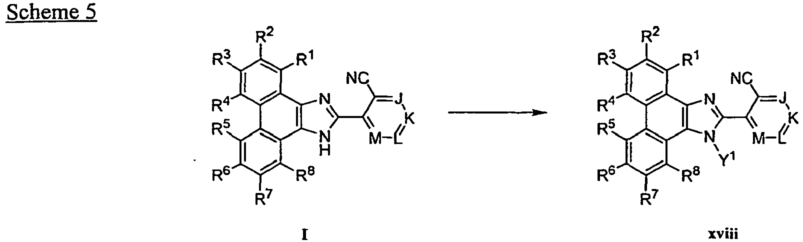

### EXAMPLES

The invention is exemplified by the following non-limiting examples:

### EXAMPLE 14

### 2-[9-chloro-6-(3-hydroxy-3-methylbutyl-1-yn-1-yl)-1H-phenanthro[9,10-d]imidazol-2-yl]-3-fluorobenzonitrile

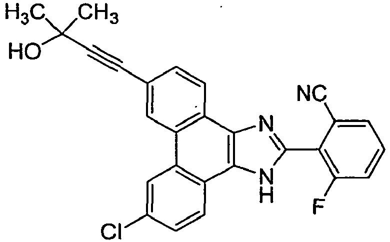

### Step 1: 6,9-dibromo-2-(2-chloro-6-fluorophenyl)-1H-phenanthro[9,10-d]imidazole

To a solution of 30 g (82 mmol) of 3,6-dibromophenanthrene-9,10-dione (Bhatt, Tetrahedron, 1963, 20, 803) in 1.0 L of acetic acid was added 25.9 g (328 mmol) of NH₄HCO₃ followed by 26 g (164 mmol) of 2-fluoro-6-chlorobenzaldehyde. The solution was stirred overnight at 130 °C, cooled down to room temperature and poured into 2.5 L of water. The mixture was filtered, washed with water followed by hexane and diethyl ether. The resulting solid was refluxed in 1.0 L of toluene with a Dean-stark apparatus and approx. 100 mL of water was removed over 3 hrs. Upon cooling down to room temperature, a beige solid crystallized out of solution. This solid was filtered, washed with toluene and pumped under reduced pressure to afford 32 g (80%) of 6,9-dibromo-2-(2-chloro-6-fluorophenyl)-1*H-*phenanthro[9,10-*d*]imidazole.

### Step 2: 2-(6-bromo-9-chloro-1H-phenanthro[9,10-d]imidazol-2-yl)-3-fluorobenzonitrile

To a DMF (10 mL) solution of 3.0 g 6,9-dibromo-2-(2-chloro-6-fluorophenyl)-1*H-*phenanthro[9,10-*d*]imidazole from Step 1, was added 587 mg of CuCN and the solution was stirred overnight at 130 °C. The solution was cooled down to room temperature followed by the addition of aqueous ammonium hydroxide and ethyl acetate. Layers were separated and the organic layer was washed with brine, dried over sodium sulphate and volatiles were removed under reduced pressure. The residue was purified by flash chromatography on silica gel using a gradient of 30 % to 50 % ethyl acetate/hexane to afford 500 mg of 2-(6-bromo-9-chloro-1*H*-phenanthro[9,10-*d*]imidazol-2-yl)-3 fluorobenzonitrile.

### Step 3: 2-[9-chloro-6-(3-hydroxy-3-methylbutyl-1-yn-1-yl)-1H-phenanthro[9,10-d]imidazol-2-yl]-3-fluorobenzonitrile

To a DMF (2 mL) solution of 2-(6-bromo-9-chloro-1*H*-phenanthro[9,10-*d*]imidazol-2-yl)-3-fluorobenzonitrile (320 mg) from Step 2 was added 5 mL of triethylamine, 0.1 mL of 2-methyl-3-butyn-2-ol, 20 mg of CuI and 82 mg of Pd(PPh₃)₄. The resulting mixture was stirred overnight at 80 °C, cooled down to room temperature and diluted with ethyl acetate/water. The organic layer was washed with brine, dried over sodium sulphate and the volatiles were removed under reduced pressure. The residue was purified by flash chromatography on silica gel using a gradient of 30 % to 50 % ethyl acetate/hexane to afford 85 mg of 2-[9-chloro-6-(3-hydroxy-3-methylbutyl-1-yn-1-yl)-1*H-*phenanthro[9,10-*d*]imidazol-2-yl]-3-fluorobenzonitrile. ¹H NMR (Acetone-d₆): δ 8.89 (s, 2H), 8.71 (bs, 1H), 8.51 (bs, 1H), 7.93 (d, 1H), 8.88-8.72 (m, 4H), 4.55 (s, 1H), 1.65 (s, 6H).

### EXAMPLE 25

### 2-(6-chloro-1H-phenanthro[9,10-d]imidazol-2-yl)isophthalonitrile

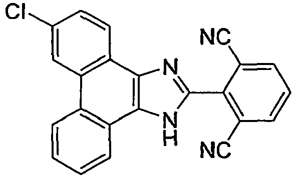

### Step 1: 1-(3-phenanthryl)ethanone oxime

In 200 mL of absolute ethanol was combined a mixture of 50 g (0.23 mol) of 1-(3-phenanthryl)ethanone and 40 g of hydroxylamine hydrochloride. The solution was heated to reflux followed by the addition of 70 mL of pyridine. After 3 hrs, the reaction was cooled down to room temperature and the solution rotovaped down. A mixture of ice/water was added to the residue and the mixture was stirred for 1 hr. The resulting off-white solid was filtered, washed with water and air dried to afford, after recristallization in diethyl ether, 32 g of 1-(3-phenanthryl)ethanone oxime.

### Step 2: 3-phenanthrylamine

To 385 g of polyphosphoric acic at 100 °C was added 32 g (0.14 mol) of 1-(3-phenanthryl)ethanone oxime from Step 1 over 30 minutes. The mixture was stirred at 100 °C for 2 hrs, cooled down to room temperature followed by the addition of water/ice. Stirred 30 minutes, filtered and washed with water. This white solid was then placed in 500 mL of methanol and 40 mL of concentrated HCl. The reaction was refluxed overnight, cooled down to room temperature and concentrated down. A mixture of ethyl acetate/water was added to the residue and the resulting solution was made basic with 10 N KOH. The aqueous layer was extracted with ethyl acetate and combined organic layers were washed with water, brine, dried over sodium sulphate and volatiles were removed under reduced pressure to afford 25 g of 3-phenanthrylamine as a beige solid.

### Step 3: 3-chlorophenanthrene

CuCl₂ (21 g) was dried under high vacuum at 115 °C for 90 minutes then cooled down to 65 °C followed by the addition of 250 mL of dry acetonitrile and 26 g of *t*-butyl nitrite. The 3-phenanthrylamine (25 g) from Step 2 was added over 30 minutes as a solution in 100 mL of acetonitrile. The reaction was stirred 45 minutes at 65 °C, cooled down to room temperature followed by the addition of 1 L of 1 N HCl. The aqueous layer was extracted with methylene chloride and combined organic layers were washed with water, brine, dried over sodium sulphate and volatiles were removed under reduced pressure. The residue was purified by flash chromatography on silica gel using hexane as the eluent to afford a white solid which was recristallized from hexane to produce 14.4 g of 3-chlorophenanthrene as a white solid.

### Step 4: 3-chlorophenanthrene-9,10-dione

To a solution of 12.5 g (58.7 mmol) of 3-chlorophenanthrene from Step 3 in 350 mL of acetic acid was added 23.5 g (0.23 mol) of CrO3. The reaction was stirred 2 hrs at 100 °C, cooled down to room temperature and poured into 2 L of water. The suspension was stirred 1 hr, filtered and washed with water. The residue was dried under high vacuum to afford 12.5 g (88%) of 3-chlorophenanthrene-9,10-dione.

### Step 5: 6-chloro-2-(2,6-dibromophenyl)-1H-phenanthro[9,10-d]imidazole

This imidazole was prepared by following the procedure describe in Example 14, Step 1, but substituting 3-chlorophenanthrene-9,10-dione for 3,6-dibromophenanthrene-9,10-dione and substituting 2,6-dibromobenzaldehyde for 2-fluoro-6-chlorobenzaldehyde to afford 27 g of 6-chloro-2-(2,6-dibromophenyl)-1*H*-phenanthro[9,10-*d*]imidazole as an off-white solid.

### Step 6: 2-(6-chloro-1H-phenanthro[9,10-d]imidazol-2-yl)isophthalonitrile

To a DMF (300 mL) solution of 32 g (65.7 mmol) of 6-chloro-2-(2,6-dibromophenyl)-1*H*-phenanthro[9,10-*d*]imidazole from Step 5 was added 14.7 g of CuCN. The reaction was stirred overnight at 80 °C, cooled down to room temperature, poured into a mixture of 1.5 L of water, 1.5 L of ethyl acetate and 200 mL of concentrated ammonium hydroxide and stirred 1 hr at room temperature. The aqueous layer was extracted with ethyl acetate and the combined organic layers were washed with 10 % ammonium hydroxide, water, brine, dried over sodium sulphate and volatiles were removed under reduced pressure. The residue was swished in toluene (2X 200 mL) and ethyl acetate (1 L). The obtained solid was purified by flash chromatography on silica gel in 5 portions using a gradient of 60% to 80% to 100% of ethyl acetate/hexane to afford 19.9 g of 2-(6-chloro-1*H*-phenanthro[9,10-*d*]imidazol-2-yl)isophthalonitrile as a pale yellow solid. ¹H NMR (400 MHz, DMSO): δ 14.32 (s, 1H), 9.0-8.9 (m, 2H), 8.55-8.45 (m, 4H), 7.99 (t, 1H), 7.85-7.78 (m, 2H), 7.72 (t, 1H).

### EXAMPLE 36

### 2-(6-bromo-9-chloro-1H-phenanthro[9,10-d]imidazol-2-yl)isophthalonitrile

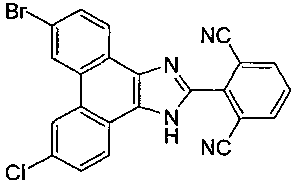

### Step1: 1-bromo-4-[2-(4-chlorophenyl)vinyl]benzene

To a solution of (4-bromobenzyl)triphenylphosphonium bromide (396 g; 0.77 mol) in 2.5 L of DMF at 0 °C, was added 37g (0.92 mol) ofNaH (60 % in oil) in four portions. The solution was stirred 1 hr at 0 °C followed by the addition of 109 g (0.77 mol) of 4-chlorobenzaldehyde in two portions. This mixture was warmed up to room temperature, stirred 1 hr and quench by pouring the reaction into a 5 °C mixture of 10 L of water and 2.5 L of Et2O. Aqueous layer was extracted with Et₂O, combined organic layers were washed with brine and dried over Na₂SO₄. Volatiles were removed under reduced pressure and the residue was dissolved in 1.5 L of cyclohexane and filtered through a pad of silica gel (wash with cyclohexane). 16 g of one isomer cristallized out of the solution as a white solid and after evaporation of the volatiles, 166 g of the other isomer 1-bromo-4-[2-(4-chlorophenyl)vinyl)benzene was isolated.

### Step 2: 3-bromo-6-chlorophenanthrene

A 2 L vessel equipped with a pyrex inner water-cooled jacket was charged with 5.16 g (17 mmol) of 1-bromo-4-[2-(4-chlorophenyl)vinyl]benzene from Step 1, 2 L of cyclohexane, 25 mL of THF, 25 mL of propylene oxide and 6.7 g (26 mmol) of iodine. The stirring solution was degassed by bubbling nitrogen and was exposed to UV light for 24 hrs by inserting a 450 W medium pressure mercury lamp in the inner. The reaction was quenched with 10% Na₂S₂O₃ and aqueous layer was extracted with ethyl acetate. Combined organic layers were washed with brine, dried over Na₂SO₄ and volatiles were removed under reduced pressure. The residue was swished in a minimal amount of ethyl acetate to afford approx. 5 g of 3-bromo-6-chlorophenanthrene as a solid.

### Step 3: 3-Bromo-6-chlorophenanthrene-9,10-dione

To a solution of 3-bromo-6-chlorophenanthrene from Step 2 (1.71 g; 5.86 mmol) in 35 mL of acetic acid was added 2.3 g (23.5 mmol) of CrO₃. The mixture was stirred 2 hrs at 100 °C, cooled down to room temperature, poured into 300 mL of water and stirred for 1 hr. The suspension was filtered, washed with water and Et₂O and pumped under reduced pressure to afford 1.67 g of 3-bromo-6-chlorophenanthrene-9,10-dione as a solid.

### Step 4: 9-bromo-6-chloro-2-(2,6-dibromophenyl)-1H-phenanthro[9,10-d]imidazole

To a solution of 15.5 g of 3-bromo-6-chlorophenanthrene-9,10-dione from Step 3 in 400 mL of acetic acid, was added 74.2 g of ammonium acetate and 19.1 g of 2,6-dibromobenzaldehyde. The mixture was stirred overnight at 120 °C, cooled down to room temperature diluted in 4 L of water and filtered. The resulting solid was refluxed 2 hrs in toluene with a Dean Stark apparatus. After cooling down to room temperature, the suspension was filtered, the solid washed with toluene and the resulting beige solid dried under high vacuum to produce 26 g of 9-bromo-6-chloro-2-(2,6-dibromophenyl)-1*H-*phenanthro[9,10-*d*]imidazole.

### Step 5: 2-(9-bromo-6-chloro-1H-phenanthro[9,10-d]imidazol-2-yl)isophthalonitrile

To a solution of 26g of 9-bromo-6-chloro-2-(2,6-dibromophenyl)-1H-phenanthro[9,10-d]imidazole from Step 4 in 200 mL of dry DMF, was added 14.2 g of CuCN. The reaction was stirred overnight at 85 °C, cooled down to room temperature, brine was added and the mixture stirred for 30 minutes. The solution was diluted in ethyl acetate, washed with 10% ammonium hydroxide, brine, dried over sodium sulphate and volatiles were removed under reduced pressure to afford 26 g of 2-(9-bromo-6-chloro-1H-phenanthro[9,10-d]imidazol-2-yl)isophthalonitrile as a solid. ¹H NMR (Acetone-d₆): 9.19 (s, 1H), 9.02 (s, 1H), 9.71 (bs, 1H), 8.49 (bs, 1H), 8.39 (d, 2H), 8.07 (t, 1H), 7.97 (d, 1H), 8.81 (d, 1H).

### EXAMPLE 40

### 2-[9-chloro-6-(3-hydroxy-3-methylbut-1-yn-1-yl)- 1Hphenanthro[9,10-d]imidazol-2-yl]isophthalonitrile

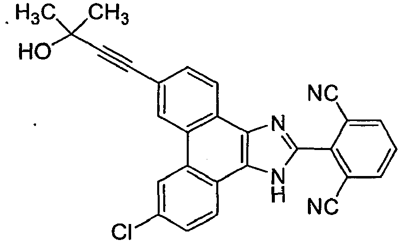

### Step1: (2E)-2-(4-bromophenyl)-3-(4-chloro-2-nitrophenyl)acrylic acid

A 2 L flask equipped with a mechanical stirrer was charged with 183 g of 2-nitro-4-chlorobenzaldehyde, 212 g of 4-bromophenylacetic acid and 233 mL of acetic anhydride. To this solution was added 82 g of potassium carbonate and the reaction was stirred overnight at 100 °C. The resulting dark mixture was cooled down to room temperature and 1.6 L of water was added followed by 800 mL of 10% HCl. The solution was decanted and taken up in water/ethyl acetate. Layers were separated, organic phase was washed with brine, dried over magnesium sulphate and volatiles were removed under reduced pressure. The residue was triturated in EtOH and the mother liquor was triturated 4 more times with EtOH to afford 219g of the desired (2*E*)-2-(4-bromophenyl)-3-(4-chloro-2-nitrophenyl)acrylic acid.

### Step 2: (2E)-3-(2-amino-4-chlorophenyl)-2-(4-bromophenyl)acrylic acid

To a 50 °C solution of 135 g of (2*E*)-2-(4-bromophenyl)-3-(4-chloro-2-nitrophenyl)acrylic acid from Step 1 in 1.2 L of acetic acid and 80 mL of water, was added 98 g of iron (powder) portion wise maintaining the temperature below 50 °C. The mixture was stirred 2 hrs at 50 °C, cooled down to room temperature, diluted with ethyl acetate (1 L) and filtered through a plug of celite. Water (1 L) was added, the layers were separated and the organic layer was washed 2 times with water, brine, dried over magnesium sulphate and volatiles were removed under reduced pressure. Residual acetic acid was removed by the addition of 1L of H₂O to the crude mixture, the solution was filtered and washed with an additional 1 L of H₂O and finally the solid was dried under high vacuum to afford 130 g of (2*E*)-3-(2-amino-4-chlorophenyl)-2-(4-bromophenyl)acrylic acid.

### Step 3: 3-Bromo-6-chlorophenanthrene-9,10-dione

This quinone can be obtained by following the procedure describe in Example 36, Step 1 to 3, or by the using the following procedure: to a 0 °C solution of 118 mL of concentrated sulphuric acid in 1.0 L of water was added drop wise a solution prepared as follows: 65 g of (2*E*)-3-(2-amino-4-chlorophenyl)-2-(4-bromophenyl)acrylic acid from Step 2 in 1 L of water followed by the addition of 11 g of NaOH, stirring for 10 minutes at 0 °C, addition of NaNO₂ (15 g) and stirring of the resulting solution at 0 °C for 20 minutes. After 30 minutes, sulfamic acid (12.5 g) was added to this mixture and after the gaz evolution seized, 1.3 L of acetone was added and the solution was stirred at 0 °C for 10 minutes. This mixture was then added to a solution of ferrocene (6.9 g) in 480 mL of acetone resulting in the formation of a green precipitate. After stirring for 20 minutes, water (2.0 L) was added, the solid was filtered and the 6-bromo-3-chlorophenanthrene-9-carboxylic acid was obtained and allowed to air dry. This crude phenanthrene was placed in 2.0 L of acetic acid followed by the addition of 54 g of CrO₃. The reaction was placed at 110 °C and after stirring for 1 hr, 18 g of CrO₃ were added. The reaction was monitored by TLC and 18 g of CrO₃ were added every hour for 3 hours where 100% conversion was observed by ¹H NMR. The mixture was cooled to room temperature, diluted in water (2.0 L), filtered and washed with water (1.0 L) to afford, after drying, 37 g of 3-Bromo-6-chlorophenanthrene-9,10-dione as a yellow solid.

### Step 4: 9-bromo-6-chloro-2-(2,6-dibromophenyl)-1H-phenanthro[9,10-d]imidazole

This imidazole was obtained following the procedure describe for Example 36, Step 4.

### Step 5: 2-(9-bromo-6-chloro-1H-phenanthro[9,10-d]imidazol-2-yl)isophthalonitrile

This imidazole was obtained following the procedure describe for Example 36, Step 5.

### Step6: 2-[9-chloro-6-(3-hydroxy-3-methylbut-1-yn-1-yl)-1H-phenanthro[9,10-d]imidazol-2-yl]isophthalonitrile

To a solution of 13 g of 2-(9-bromo-6-chloro-1*H*-phenanthro[9,10-*d*]imidazol-2-yl)isophthalonitrile in 240 mL of DMF is added 5.5 mL of 2-methyl-3-butyn-2-ol, 2.0 g of tetrakis(triphenylphosphine)palladium, 1.1 g of copper iodide and 5.6 mL of diisopropylamine. The mixture is stirred at 55 °C for 1 hr then cooled to room temperature and diluted with ethyl acetate (250 mL). Water (250 mL) is added and the layers were separated, the organic phase is washed with brine, dried over magnesium sulphate and volatiles are removed under reduced pressure. The crude mixture is then purified on silica gel using 50% hexane/ethyl acetate. The product is then recrystallized in THF and triturated in hot ethyl acetate/ether mixture to afford 5.4 g of [9-chloro-6-(3-hydroxy-3-methylbut-1-yn-1-yl)-1*H*-phenanthro[9,10-*d*]imidazol-2-yl]isophthalonitrile as a light yellow solid. ¹H NMR (Acetone-d₆): 8.93 (s, 2H), 8.53 (m, 2H), 8.36 (d, 2H), 8.01 (t, 1H), 7.78 (d, 2H), 4.53 (s, 1H), 1.61 (s, 6H).

### EXAMPLE 60

### 2-(1-{[dihydroxy(dioxido)phosphino]methyl}-1H-phenanthro[9,10-d]imidazol-2-yl)isophthalonitrile

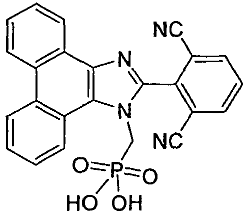

### Step 1: 2-(2,6-dibromophenyl)-1H-phenanthro[9,10-d]imidazole

This imidazole was obtained following the procedure described in Example 36, Step 4, but substituting the phenanthrene-9,10-dione for the 3-bromo-6-chlorophenanthrene-9,10-dione to afford the 2-(2,6-dibromophenyl)-1*H*-phenanthro[9,10-*d*]imidazole

### Step 2: 2-(1H-phenanthro[9,10-d]imidazol-2-yl)isophthalonitrile

This compound was obtained by using the procedure described in Example 36, Step 5, but substituting the 2-(2,6-dibromophenyl)-1*H*-phenanthro[9,10-d]imidazole for the 9-bromo-6-chloro-2-(2,6-dibromophenyl)-1*H*-phenanthro[9,10-*d*]imidazole to afford the desired 2-(1*H*-phenanthro[9,10-*d*]imidazol-2-yl)isophthalonitrile.

### Step 3: 2-[1-(chloromethyl)-1H-phenanthro[9,10-d]imidazol-2-yl]isophthalonitrile

2-(1*H*-phenanthro[9,10-*d*]imidazol-2-yl)isophthalonitrile from Step 2 (1 g, 2.91 mmol) was mixed with cesium carbonate (1.14 g, 3.49 mmol) in chloroiodomethane (10 mL). The mixture was heated to 80°C overnight. The reaction was cooled to room temperature and poured into 200 mL water and 500 mL ethyl acetate. The layers were separated, and the organic layer was washed with 200 mL water, 200 mL saturated aqueous sodium bicarbonate solution, 100 mL brine, and dried over anhydrous magnesium sulfate. The solvent was removed under reduced pressure. The crude solid was purified by flash column chromatography using 40% ethyl acetate in hexane to give 357 mg of 2-[1-(chloromethyl)-1*H*-phenanthro[9,10-*d*]imidazol-2-yl]isophthalonitrile (31%) plus 650 mg of a mixture of product and starting material.

### Step 4: 2-(1-{[dihydroxy(dioxido)phosphino]methyl}-1H-phenanthro[9,10-d]imidazol-2-yl)isophthalonitrile

The 2-[1-(chloromethyl)-1*H*-phenanthro[9,10-*d*]imidazol-2-yl]isophthalonitrile from Step 3 (200 mg, 0.509 mmol) was mixed with tetramethylammonium di(*tert*-butyl)phosphate (288 mg, 1.02 mmol) in DMF (5 mL) and heated at 50°C for 8 hours. It was cooled to room temperature and poured into 15 mL water and 35 mL ethyl acetate. The layers were separated, and the organic layer was washed with 10 mL water (twice), 10 mL saturated aqueous sodium bicarbonate solution, brine, and dried over anhydrous magnesium sulfate. The solvent was removed under reduced pressure. The crude solid was purified by flash column chromatography using 50-70% ethyl acetate in hexane to give 221 mg of protected phosphate (77%). 155 mg of this solid was dissolved in 10% TFA/toluene (3 mL) and stirred at room temperature overnight. The solvent was removed under reduced pressure. The resulting crude product was purified by a semi-preparative RP-HPLC using a C 18 column and eluting with a gradient of 44-49% acetonitrile + 0.2% TFA over 8 min. The fractions containing product were combined and lyophilized to give 80 mg of the desired 2-(1-{[dihydroxy(dioxido)phosphino]methyl}-1*H-*phenanthro[9,10-*d*]imidazol-2-yl)isophthalonitrile.
¹H NMR (DMSO): 9.05 (d, 1H), 8.95 (d, 1H), 8.54-8.61 (m, 2H), 8.47 (d, 2H), 8.06 (t, 1H), 8.70-8.85 (m, 4H), 6.21 (d, 2H).

### EXAMPLE 87

### 2-[6-bromo-9-(1-hydroxy-1-methylethyl)-1H-phenanthro[9,10-d]imidazol-2-yl]isophthalonitrile

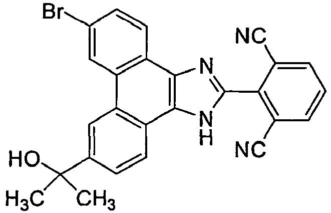

### Step 1: 6,9-dibromo-2-(2,6-dibromophenyl)-1H-phenanthro[9,10-d]imidazole

A suspension of di-bromoquinone (38.6 g, 0.1 mol), ammonium acetate (165 g, 2.1 mol) and dibromobenzaldehyde (45 g, 0.1 mol) in acetic acid (1.5 L) was heated at reflux for 16 h. The reaction mixture was quenched by pouring it into water (2.2 L), followed by stirring for 2 h. The resulting solid was filtered and rinsed successively with water and hexanes. The solids were then heated at reflux in toluene (600 mL) with a Dean Stark for 4h and then filtered to afford the desired 6,9-dibromo-2-(2,6-dibromophenyl)-1*H*-phenanthro[9,10-d]imidazole as a beige powder (62.3 g, 97%).

### Step 2: 6,9-dibromo-2-(2,6-dibromophenyl)-1-{[2-(trimethylsilyl)ethoxy]methyl}-1H-phenanthro[9,10-d]imidazole

To a suspension of 6,9-dibromo-2-(2,6-dibromophenyl)-1*H*-phenanthro[9,10-*d*]imidazole from Step 1 (61.8g, 0.1 mol) in THF (980 mL) at 0 ^{o}C, was added sodium hydride (60% dispersion in mineral oil, 10 g, 0.25 mol). The suspension was stirred at 0 ^{o}C for 15 minutes, followed by addition of SEMCI (45 mL, 0.25 mol). The mixture was warmed to room temperature and stirred for 3 h, after which it was poured into water. The aqueous phase was extracted with ethyl acetate, the organic layer washed once with brine, dried over Na₂SO₄, filtered and concentrated. The crude material was swished in hexanes/diethyl ether for 4h, then filtered to obtain 6,9-dibromo-2-(2,6-dibromophenyl)-1-{[2-(trimethylsilyl)ethoxy]methyl}-1*H*-phenanthro[9,10-*d*]imidazole as a beige powder (71.5 g, 95 %).

### Step 3: methyl 6-bromo-2-(2,6-dibromophenyl)-1-[2-(trimethylsilyl)ethoxy]methyl}-1H-phenanthro[9,10-d]imidazole-9-carboxylate

To a solution of 6,9-dibromo-2-(2,6-dibromophenyl)-1-{[2-(trimethylsilyl)ethoxy]methyl}-1*H*-phenanthro[9,10-*d*]imidazole from Step 2 (22.8 g, 30.8 mmol) in DMF (150 mL) and MeOH (150 mL) in a 3-necked 1 L round-bottomed flask, was added Pd(OAc)₂ (350 mg, 1.5 mmol) and dppf (1.7 g, 3.0 mmol). The mixture was degassed three times and back-filled with carbon monoxide. Triethylamine (9.5 mL, 43 mmol) was then added and the reaction mixture was heated at 60 ^{o}C, under an atmosphere of carbon monoxide, for 1 h. The reaction was quenched by pouring it into water and ethyl acetate. It was then filtered through Celite, the aqueous phase extracted with ethyl acetate, the organic layer washed once with brine, dried over Na₂SO₄, filtered and concentrated. The crude material was purified by flash chromatography on silica (0-5 % ethyl acetate in toluene) to afford the isomers of the desired methyl 6-bromo-2-(2,6-dibromophenyl)-1-{[2-(trimethylsilyl)ethoxy]methyl}-1*H*-phenanthro[9,10-*d*]imidazole-9-carboxylate as beige solids (9.8 g, 44%).

### Step 4: 2-[6-bromo-2-(2,6-dibromophenyl-1H-phenanthro[9,10-d]imidazol-9-yl]propan-2-ol

To a -78 ^{o}C solution of isomeric methyl 6-bromo-2-(2,6-dibromophenyl)-1-{[2-(trimethylsilyl)ethoxy]methyl}-1*H*-phenanthro[9,10-*d*]imidazole-9-carboxylate from Step 3 (9.9 g, 13.8 mmol) in CH₂Cl₂ (200 mL) was added methyl magnesium bromide (3.0 M in Et₂O, 33 mL) via addition funnel. The mixture was then warmed to -40 ^{o}C, stirred at this temperature for 0.5 h, then warmed to between -30 and -35 ^{o}C and stirred at this temperature for 2 h. The reaction mixture was then warmed to -25 ^{o}C, stirred for 3 h, and then stirred at 0 ^{o}C for 1.5 h. The reaction was quenched by pouring it into water and ethyl acetate. The aqueous phase was extracted with ethyl acetate, the organic layer washed once with brine, dried over Na₂SO₄, filtered and concentrated. The crude product was dissolved in THF (150 mL) and cooled to 0 ^{o}C. TBAF (1.0 M in THF, 35 mL) was then added and the mixture heated at reflux for 17 h, then quenched with 25 % NH₄OAc, the aqueous phase extracted with ethyl acetate, the organic layer washed once with brine, dried over Na₂SO₄, filtered and concentrated. The material obtained after purification by flash chromatography on silica (5-30 % THF in toluene) was swished in toluene for 5 h and then filtered to afford 2-[6-bromo-2-(2,6-dibromophenyl)-1*H*-phenanthro[9,10-*d*]imidazol-9-yl]propan-2-ol as a white powder (4.53 g, 56 %, 2 steps).

### Step 5: 2-[6-bromo-9-(1-hydroxy-1-methylethyl)-1H-phenanthro[9,10-d]imidazol-2-yl]isophthalonitrile

Copper cyanide (420 mg, 4.7 mmol) was added to a room temperature solution of 2-[6-bromo-2-(2,6-dibromophenyl)-1*H*-phenanthro[9,10-*d*]imidazol-9-yl]propan-2-ol from Step 4 (1.25 g, 2.1 mmol) in DMF (100 mL) and the mixture heated at 80 ^{o}C for 18 h, after which it was poured into a mixture of NH₄OH and ethyl acetate and stirred for 1 h. The aqueous phase was extracted with ethyl acetate, the organic layer washed once with water, once with brine, dried over Na₂SO₄, filtered and concentrated. The material obtained after purification by flash chromatography on silica (20-80% ethyl acetate in toluene) was swished in ethyl acetate and THF for 2 h and then filtered to afford 2-[6-bromo-9-(1-hydroxy-1-methylethyl)-1*H*-phenanthro[9,10-d]imidazol-2-yl]isophthalonitrile as a yellow solid (250 mg, 25%).
1H NMR δ (ppm)(DMSO with added TFA): 9.08 (1 H, s), 8.90 (1 H, s), 8.45-8.39 (4 H, m), 7.99-7.91 (3 H, m), 1.61 (6 H, s).

### EXAMPLE 88

### 2-[6-(cyclopropylethynyl)-9-(1-hydroxy-1-methylethyl)-1H-phenanthro[9,10-d]imidazol-2-yl]isophthalonitrile

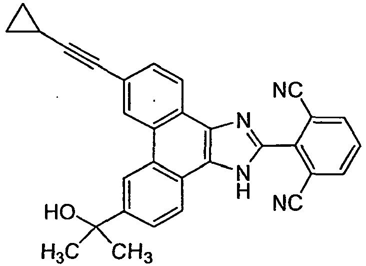

### Step 1: 2-[6-(cyclopropylethynyl)-9-(1-hydroxy-1-methylethyl)-1H-phenanthro[9,10-d]imidazol-2-yl]isophthalonitrile

A round bottomed flask containing 2-[6-bromo-9-(1-hydroxy-1-methylethyl)-1*H-*phenanthro[9,10-d]imidazol-2-yl]isophthalonitrile from Example 87(1.26 g, 2.62 mmol), Pd(PPh₃)₄ (190 mg, 0.27 mmol) and copper iodide (100 mg, 0.52 mmol) was purged with nitrogen for 15 minutes, followed by addition of DMF (50 mL), cyclopropyl acetylene (1.4 mL, 21 mmol) and di-isopropylamine (560 µL, 4 mmol). The resulting mixture was heated at 60-65 ^{o}C for 3.5 h, cooled to room temperature and then poured into a mixture of NH₄OH and ethyl acetate and stirred for 1 h. The aqueous phase was extracted with ethyl acetate, the organic layer washed once with water, once with brine, dried over Na₂SO₄, filtered and concentrated. The material obtained after purification by flash chromatography on silica (30-100% ethyl acetate in toluene) was swished in toluene for 2 h and then filtered to afford 2-[6-(cyclopropylethynyl)-9-(1-hydroxy-1-methylethyl)- 1*H*-phenanthro[9,10-*d*]imidazol-2-yl]isophthalonitrile as a yellow solid (350 mg). The mother liquor was combined with the mixed fractions and re-purified by flash chromatography on silica (3-40% acetonitrile in toluene) to afford 286 mg the bis-nitrile (total yield 52%).
1H NMR δ (ppm)(DMSO with added TFA): 8.92 (1 H, s), 8.87 (1 H, s), 8.43-8.39 (4 H, m), 7.96 (1 H, t), 7.90 (1 H, d), 7.71 (1 H, d), 1.60 (7 H, s), 0.90 (2 H, t), 0.84 (2 H, d).

### EXAMPLE 117

### 2-[9-chloro-6-(3-hydroxy-3-methylbutyl)-1H-phenanthro[9,10-d]imidazol-2-yl)isophthalonitrile

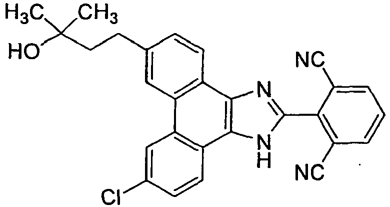

### Step 1: 2-[9-chloro-6-(3-hydroxy-3-methylbutyl)-1H-phenanthro[9,10-d]imidazol-2-yl)isophthalonitrile

To a solution of 9-BBN in THF (24 ml, 12 mmol, 0.5 M) was added 2-methyl-3-buten-2-ol (345 mg, 4.0 mmol) and the resulting solution was stirred under N₂ at rt for overnight. In a second flask charged with PdCl₂(dppf) (324 mg, 0.40mmol), Cs₂CO₃ (2.4 g, 8.0 mmol) and Ph₃As (124 mg, 0.4 mmol) was added 2-(6-bromo-9-chloro-1*H*-phenanthro[9,10-*d*]imidazol-2-yl)isophthalonitrile from Example 36, DMF (24 ml) and H₂O (0.88 ml) and the mixture was stirred under N₂ for 5 minutes. The hydroboration mixture was then transferred to the second flask and the resulting reaction suspension was stirred at rt under N₂ for 5 days. After being treated with brine, the aqueous phase was extracted with EtOAc and the combined organic solution was washed with water and brine, dried over MgSO₄. After removing the drying agent by filtration, the solution was concentrated under reduced pressure and the residue was purified by silica gel chromatography (50% EtOAc/Hexane) to yield 600 mg of 2-[9-chloro-6-(3-hydroxy-3-methylbutyl)-1*H*-phenanthro[9,10-d]imidazol-2-yl)isophthalonitrile as a yellow solid. ¹H NMR (400 MHz, Acetone): δ 13.10 (s br, 1H); 8.94 (s, 1 H); 8.77 (s, 1 H); 8.70-8.60 (m br, 2 H); 8.39 (d, 2 H); 8.03 (t, 1 H); 7.75 (dd, 1 H); 7.69 (dd, 1 H); 4.92 (s, 1 H); 3.05 (m, 2 H); 1.95 (m, 2 H); 1.33 (s, 6 H).

### EXAMPLE 123

### (±)-2-[9-chloro-6-(3,4-dihydroxy-3-methylbut-1-yn-1-yl)-1H-phenanthro[9,10-d]imidazol-2-yl]isophthalonitrile

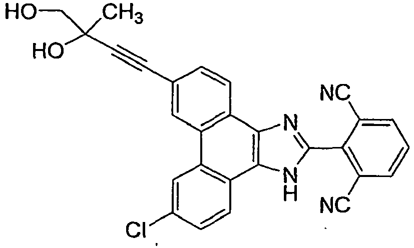

### Step 1: 2-[6-chloro-9-(3-methylbut-3-en-1-yn-1-yl)-1H-phenanthro[9,10-d]imidazol-2-yl]isophthalonitrile

To a stirred suspension of 2-[9-chloro-6-(3-hydroxy-3-methylbut-1-yn-1-yl)-1*H-*phenanthro[9,10-*d*]imidazol-2-yl]isophthalonitrile from Example 40 (120 mg, 0.26 mmol) in benzene (4 mL) was added Burgess Reagent (70 mg, 0.29 mmol) and refluxed for 2 hours under N₂. The resulting reaction mixture was diluted with EtOAc (20 mL). This EtOAc solution was washed with water, brine and dried over MgSO₄. After removing the drying agent via filtration, the organic solution was concentrated under reduced pressure. The residue was purified by silica gel column chromatography (eluted with 50/50 EtOAc/hexane) to yield 90 mg of 2-[6-chloro-9-(3-methylbut-3-en-1-yn-1-yl)-1*H-*phenanthro[9,10-*d*]imidazol-2-yl)isophthalonitrile as a yellow solid.

### Step 2: (±)-2-[9-chloro-6-(3,4-dihydroxy-3-methylbut-1-yn-1-yl)-1H-phenanthro[9,10-d]imidazol-2-yl] isophthalonitrile

To a stirred suspension of 2-[6-chloro-9-(3-methylbut-3-en-1-yn-1-yl)-1*H-*phenanthro[9,10-*d*]imidazol-2-yl]isophthalonitrile from Step 1 (22 mg, 0.05 mmol) in 50/50 *t-*BuOH/H₂O (0.5 mL) was added AD-mix-α (70 mg) at 0 ^{o}C. The mixture was left stirring at 0 ^{o}C for 24 hours. The resulting reaction mixture was treated with saturated Na₂S₂O₃ aqueous solution and stirred for 10 minutes, diluted with water and extracted with EtOAc. This EtOAc solution was washed with water, brine and dried over MgSO₄. After removing the drying agent via filtration, the organic solution was concentrated under reduced pressure. The residue was purified by silica gel column chromatography (eluted with 50/50 EtOAc/hexane to 95/5 EtOAc/MeOH) to yield 19 mg of yellow solid. This same procedure was repeated with AD-mix-β to yield another 19 mg of yellow solid. These two yellow solids were combined to give the racemic 2-[9-chloro-6-(3,4-dihydroxy-3-methylbut-1-yn-1-yl)-1*H-*phenanthro[9,10-*d*]imidazol-2-yl]isophthalonitrile.
¹H NMR (400 MHz, Acetone): δ 8.84 (d, 1 H); 8.80 (s, 1 H); 8.57 (d, 1 H); 8.47 (d, 1 H); 8.39 (d, 2 H); 8.03 (t, 1 H); 7.77 (dd, 8.6 Hz, 1 H); 7.71 (dd, 1 H); 4.56 (s, 1 H); 4.30 (s, 1 H); 3.67 (q, 2 H); 1.56 (s, 3 H).

### ASSAYS FOR DETERMINING BIOLOGICAL ACTIVITY

### Inhibition of prostaglandin E synthase activity

Compounds are tested as inhibitors of prostaglandin E synthase activity in microsomal prostaglandin e synthases, whole cell and in vivo assays. These assays measure prostaglandin E2 (PGE2) synthesis using either Enzymatic Immunoassay (EIA) or mass spectrometry. Cells used for microsomal preparation are CHO-K1 cells transiently transfected with plasmids encoding the human mPGES-1 cDNA. Cells used for cell-based experiments are human A549 (which express human mPGES-1). Guinea pigs are used to test the activity of selected compounds in vivo. In all these assays, 100% activity is defined as the PGE₂ production in vehicle-treated samples. IC₅₀ and ED₅₀ represent the concentration or dose of inhibitor required to inhibit PGE₂ synthesis by 50% as compared to the uninhibited control.

### Microsomal prostaglandin E synthase assay

Prostaglandin E synthase microsomal fractions are prepared from CHO-K1 cells transiently transfected with plasmid encoding the human mPGES-1 cDNA. Microsomes are then prepared and the PGES assay begins with the incubation of 5µg/ml microsomal PGES-1 with compound or DMSO (final 1%) for 20-30 minutes at room temperature. The enzyme reactions are performed in 200mM KPi pH 7.0, 2mM EDTA and 2.5mM GSH-reduced form. The enzymatic reaction is then initiated by the addition of 1µm fmal PGH₂ substrate prepared in isopropanol (3.5% final in assay well) and incubated at room temperature for 30 seconds. The reaction is terminated by the addition of SnCl₂ in IN HCl (1mg/ml final). Measurement of PGE₂ production in the enzyme reaction aliquots is done by EIA using a standard commercially available kit (Cat #: 901-001 from Assay Designs).

Data from this assay for representative compounds is shown in the table below. The potency is expressed as IC₅₀ and the value indicated is an average of at least n=3.

| Ex. | h-CHO (nM) |
|---|---|
| 1 | 1.9 |
| 5 | 2.1 |
| 8 | 2 |
| 9 | 1.9 |
| 14 | 1.8 |
| 20 | 13.1 |
| 21 | 12 |
| 25 | 1.3 |
| 23 | 2.1 |
| 36 | 1.2 |
| 37 | 9.9 |
| 40 | 0.9 |
| 45 | 2534 |
| 46 | 1.5 |
| 48 | 0.9 |
| 51 | 4.8 |
| 55 | 1.1 |
| 56 | 1.7 |
| 65 | 1.5 |
| 68 | 1.5 |
| 73 | 1.7 |
| 76 | 3.7 |
| 87 | 1.9 |
| 88 | 1.3 |
| 91 | 1 |
| 93 | 1.2 |
| 95 | 2.4 |
| 98 | 0.9 |
| 99 | 1.2 |
| 117 | 0.7 |

### Human A549 whole cell prostaglandin E synthase assay

### Rationale

Whole cells provide an intact cellular environment for the study of cellular permeability and biochemical specificity of anti-inflammatory compounds such as prostaglandin E synthase inhibitors. To study the inhibitory activities of these compounds, human A549 cells are stimulated with 10ng/ml recombinant human IL-1β for 24 hours. The production of PGE₂ and PGF_{2α}, are measured by EIA at the end of the incubation as readouts for selectivity and effectiveness against mPGES-1-dependent PGE₂ production.

### Methods

Human A549 cells specifically express human microsomal prostaglandin E synthase-1 and induce its expression following treatment with IL-1β for 24 hours. 2.5x10⁴ cells seeded in 100ul/well (96-well plate) and incubated overnight under standard conditions. 100 ul of cell culture media containing 10ng/ml IL-1β is then added to the cells followed by the addition of either 2% FBS containing RPMI or 50% FBS containing RPMI. 2µl of drugs or vehicle (DMSO) are then added and samples are mixed immediately. Cells are incubated for 24 hours and following the incubation 175µl of medium is harvested and assayed for PGE₂ and PGF_{2α}, contents by EIA.

### Human whole blood prostaglandin E synthase assay

### Rationale

Whole blood provides a protein and cell-rich milieu for the study of biochemical efficacy of anti-inflammatory compounds such as prostaglandin E synthase inhibitors. To study the inhibitory activities of these compounds, human blood is stimulated with lipopolysaccharide (LPS) for 24 hours to induce mPGES-1 expression. The production of prostaglandin E2 (PGE₂) and thromboxane B2 (TxB₂) are measured by EIA at the end of the incubation as readouts for selectivity and effectiveness against mPGES-1-dependent PGE₂ production.

### Methods

Human whole blood assays for mPGES-1 activity reported (Brideau, et al., Inflamm. Res., vol. 45, p. 68, 1996) are performed as described below.

Freshly isolated venous blood from human volunteers is collected in heparinized tubes. These subjects have no apparent inflammatory conditions and have not taken any NSAIDs for at least 7 days prior to blood collection. 250 µl of blood is pre-incubated with 1 ul vehicle (DMSO) or 1 ul of test compound. Bacterial LPS at 100µg/ml (E. Coli serotype 0111:B4 diluted in 0.1% w/v bovine serum albumin in phosphate buffered saline) is then added and samples are incubated for 24 hours at 37°C. Unstimulated control blood at time zero (no LPS) is used as blank. At the end of the 24hr incubation, the blood is centrifuged at 3000rpm for 10 min at 4°C. The plasma is assayed for PGE₂ and TxB₂ using an EIA kit as indicated above.

### In vivo determination of anti-inflammatory activity

### Rationale

The whole animal provides an integrated physiological system to confirm the anti-inflammatory activity of test compounds characterized in vitro. To determine the activity of prostaglandin E synthase inhibitors in vivo, animals are dosed with compounds either prior or after the inflammatory stimulus, LPS. LPS is injected into the hind paw of guinea pigs and hyperalgesia measurements are recorded 4.5 and/or 6 hrs after the injection.

### Methods

Male Hartley guinea pigs, weighing 200-250 grams were used. LPS (30 mg/kg) is injected sub-plantarly into the left hind paw of the guinea pig to produce hyperalgesia in the injected paw. Rectal temperature and paw withdrawal latency, a measure of hypersensitivity to pain (hyperalgesia), are taken prior to LPS injection and used as the baseline. Paw withdrawal latency is determined using the thermal hyperalgesia instrument (Ugo Basile Corp.). During this determination, animals are placed in an 8"x8" plexiglas holding box atop of a glass base. A mild (223mW/cm²) infrared light is directed toward the underside of the hind paw. The time it takes for the animal to remove its paw (indication that it feels the pain caused by the heat) is recorded. The infrared light immediately shuts off when the animal withdraws its paw from the area. The light will also shut off automatically when the time reaches 20 seconds.

### Predose paradigm:

Test compounds are orally dosed at 5ml/kg using an 18-gauge feeding needle. LPS (serotype 0111:B4, 10 µg) or 0.9% saline is injected into the plantar region of the left hind paw at a volume of 100 µl using a 26 gauge needle 1 hour following compound administration. Rectal temperature and thermal paw withdrawal latency are taken 4.5 hours after LPS administration. The animals are euthanized following the measurements using CO₂ and lumbar spinal cord, hind paw and blood samples collected.

### Reversal paradigm:

Thermal paw withdrawal of each animal is determined before and 3 hours following sub-plantar injection of LPS. Animals which have received LPS and do not show a decrease in withdrawal latency at the 3 hour time point will be removed from study and euthanized. Test compounds are dosed p.o. at 5ml/kg immediately following the thermal paw withdrawal measurement. Thermal withdrawal latency is taken 1.5 and 3hours following compound administration (4.5 and 6 hours post-LPS administration). After the final reading, the animals are euthanized using CO2 and lumbar spinal cord and blood samples collected for prostaglandin determination by mass spectrometry and drug level, respectively.

## Claims

1. A compound represented by Formula I 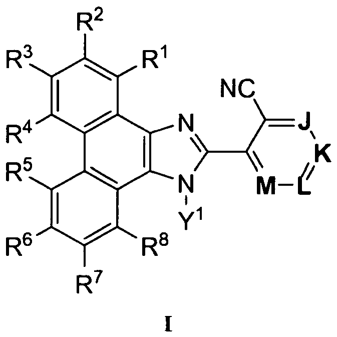 or a pharmaceutically acceptable salt of said compound, wherein:
Y¹ is H or is selected from the group consisting of: (1) C₁₋₆alkyl; (2) PO₄-C₁₋₄alkyl-; (3) C₁₋₄alkyl-C(O)-O-CH₂-, wherein the C₁₋₄alkyl portion is optionally substituted with R³³-O-C(O)-; and (4) C₁-₄alkyl-O-C(O)-;
R33 is selected from the group consisting of: (1) H; (2) C₁₋₄alkyl, (3) C₃₋₆cyloalkyl; (4) phenyl; (5) benzyl; and (6) pyridyl; said C₁₋₄alkyl, C₃₋₆cycloalkyl, phenyl, benzyl and pyridyl may each be optionally substituted with 1 to 3 substituents independently selected from the group consisting of: OH, F, Cl, Br and I;
**J** is selected from the group consisting of -C(X²)- and -N-,
**K** is selected from the group consisting of -C(X³)- and -N-,
**L** is selected from the group consisting of -C(X⁴)- and -N-, and
**M** is selected from the group consisting of -C(X⁵)- and -N-,
with the proviso that at least one of **J**, **K**, **L** or **M** is other than -N-;
X², X³, X4 and X⁵ are independently selected from the group consisting of: (1) H; (2) -CN; (3) F; (4) Cl; (5) Br; (6) I; (7) -OH; (8) -N₃; (9) C₁₋₆alkyl, C₂₋₆alkenyl or C₂₋₆alkynyl, wherein one or more of the hydrogen atoms attached to said C ₁₋₆alkyl, C₂₋₆alkenyl or C₂₋₆alkynyl may be replaced with a flouro atom, and said C₁₋₆alkyl, C₂₋₆alkenyl or C₂₋₆alkynyl may be optionally substituted with a hydroxy group; (10) C₁₋₄alkoxy; (11) NR⁹R¹⁰-C(O)-C₁₋₄alkyl-O-; (12) C₁₋₄alkyl-S(O)ₖ-; (13) -NO₂; (14) C₃-₆cycloalkyl, (15) C₃₋₆cycloalkoxy; (16) phenyl, (17) carboxy; and (18) C₁₋₄alkyl-O-C(O)-;
R¹, R2, R³, R4, R⁵, R⁶, R⁷ and R8 are independently selected from the group consisting of: (1) H; (2) F; (3) Cl; (4) Br; (5) I; (6) -CN; (7) C₁₋₆alkyl or C₂₋₆alkenyl, wherein one or more of the hydrogen atoms attached to said C₁₋₆alkyl or C₂₋₆alkenyl may be replaced with a fluoro atom, and wherein said C₁₋₆alkyl or C₂₋₆alkenyl may be optionally substituted with one to three substituents independently selected from the group consisting of: -OH, methoxy, R¹¹-O-C(O)-, cyclopropyl, pyridyl and phenyl; (8) C₃₋₆cycloalkyl; (9) R¹²-O-; (10) R¹³-S(O)ₖ-, (11) R¹⁴-S(O)ₖ-N(R¹⁵)-; (12) R¹⁶-C(O)-; (13) R¹⁷-N(R¹⁸)-; (14) R¹⁹-N(R²⁰)-C(O)-; (15) R²¹-N(R²²)-S(O)ₖ-; (16) R²³-C(O)-N(R²⁴)-; (17) Z-C≡C; (18) -(CH₃)C=N-OH or-(CH₃)C=N-OCH₃; and (19) phenyl, naphthyl, pyridyl, pyradazinyl, pyrimidinyl, pyrazinyl, pyrrolyl, pyrazolyl, imidazolyl, triazolyl, tetrazolyl, oxazolyl, isoxazolyl, oxadiazolyl, thienyl or furyl, each optionally substituted with a substituent independently selected from the group consisting of: F, Cl, Br, I, C₁₋₄alkyl, phenyl, methylsulfonyl, methylsulfonylamino, R²⁵-O-C(O)- and R26-N(R27)-, said C₁₋₄alkyl optionally substituted with 1 to 3 groups independently selected from halo and hydroxy;
each **Z** is independently selected from the group consisting of: (1) H; (2) C₁₋₆alkyl, wherein one or more of the hydrogen atoms attached to said C₁₋₆alkyl may be replaced with a flouro atom, and wherein C ₁₋₆alkyl is optionally substituted with one to three substituents independently selected from: hydroxy, methoxy, cyclopropyl, phenyl, pyridyl, pyrrolyl, R28-N(R29)- and R³⁰-O-C(O)-; (3) -(CH₃)C=N-OH or -(CH₃)C=N-OCH₃; (4) R³¹-C(O)-; (5) phenyl; (6) pyridyl or the N-oxide thereof; (7) C₃₋₆cycloalkyl, optionally substituted with hydroxy; (8) tetrahydropyranyl, optionally substituted with hydroxy; and (9) a five-membered aromatic heterocycle containing 1 to 3 atoms independently selected from O, N or S and optionally substituted with methyl;
each R⁹, R¹⁰, R¹⁵, R²⁴ and R³² is independently selected from the group consisting of: (1) H; and (2) C₁₋₄alkyl;
each R¹¹, R¹², R¹³, R¹⁴, R¹⁶, R²³, R25, R³⁰ and R³¹ is independently selected from the group consisting of: (1) H; (2) C₁₋₄alkyl, (3) C₃₋₆cycloalkyl; (4) phenyl, (5) benzyl; and (6) pyridyl; said C₁-₄alkyl, C₃₋₆cycloalkyl, phenyl, benzyl and pyridyl may each be optionally substituted with 1 to 3 substituents independently selected from the group consisting of: OH, F, Cl, Br and I;
each R¹⁷, R¹⁸, R¹⁹, R²⁰, R²¹, R²², R²⁶, R²⁷, R²⁸ and R²⁹ is independently selected from the group consisting of: (1) H; (2) C₁₋₆alkyl; (3) C₁₋₆alkoxy; (4) OH and (5) benzyl or 1-phenylethyl; and R¹⁷ and R¹⁸, R¹⁹ and R²⁰, R²¹ and R²², R²⁶ and R²⁷, and R²⁸ and R²⁹ may be joined together with the nitrogen atom to which they are attached to form a monocyclic ring of 5 or 6 carbon atoms, optionally containing one or two atoms independently selected from -O-, -S(O)ₖ- and -N(R3²)-; and
each k is independently 0, 1 or 2.

2. A compound according to Claim 1 according to Formula A 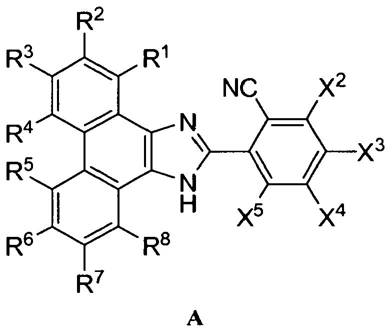 or a pharmaceutically acceptable salt of said compound.

3. The compound according to Claim 2 wherein:
X², X³, X⁴ and X⁵ are independently selected from the group consisting of: (1) H; (2) -CN; (3) F; (4) Cl; (5) Br; and (6) I.

4. The compound according to Claim 2, wherein X², X³ and X⁴ are H, and X⁵ is other than H.

5. The compound according to Claim 4, wherein X⁵ is -CN.

6. The compound according to Claim 2, wherein at least one of R¹ or R⁸ is other than H.

7. The compound according to Claim 2 wherein at least one of R² or R⁷ is other than H.

8. The compound according to Claim 2 wherein at least one of R⁴ or R⁵ is other than H.

9. The compound according to Claim 2 wherein:
at least one of R³ or R⁶ is other than H; and
R¹, R², R⁴, R⁵, R⁷ and R⁸ are H.

10. The compound according to Claim 9, wherein R³ and R⁶ are both other than H.

11. The compound according to Claim 10, wherein:
one of R³ or R⁶ is independently selected from the group consisting of: F, Cl, Br, and I; and
the other of R³ or R⁶ is **Z**-C≡C.

12. The compound according to Claim 9, wherein: R³ and R⁶ are independently selected from the group consisting of: hydrogen, fluoro, chloro, bromo, iodo, cyano, methyl, ethyl, vinyl, cyclopropyl, -CO₂*i*-Pr, -CO₂CH₃, -SO₂CF₃, 3-pyridyl, acetyl, 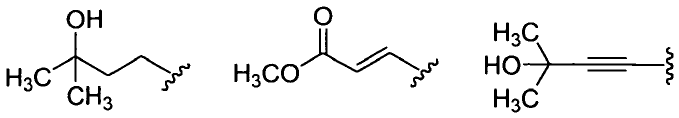 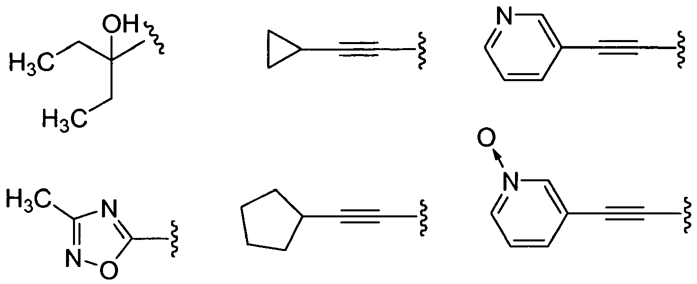 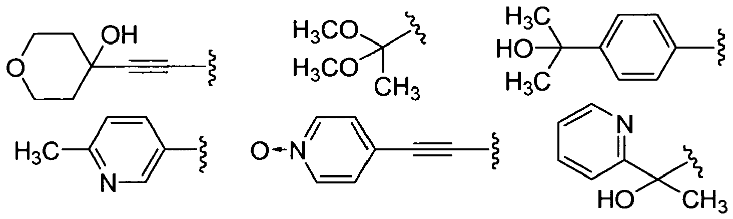 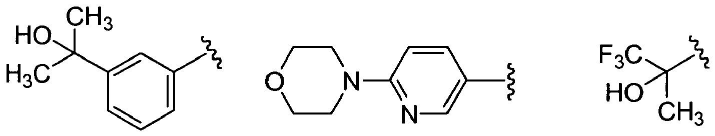 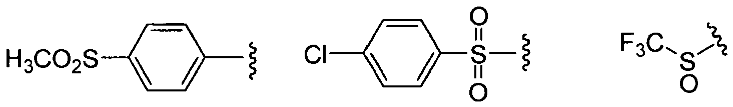 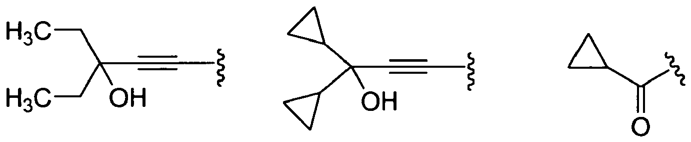 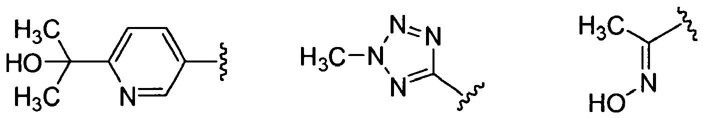 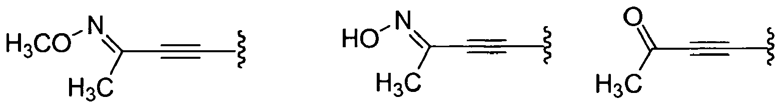 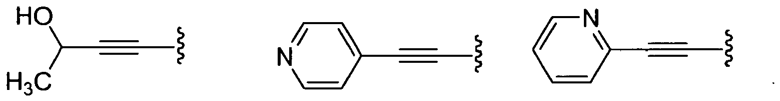 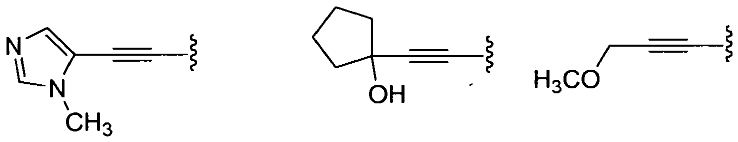 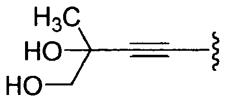 with the proviso that at least one of R³ or R⁶ is other than H.

13. The compound according to Claim 1 according to Formula **B**: 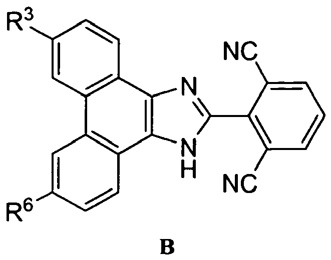 or a pharmaceutically acceptable salt of said compound.

14. The compound according to Claim 13 wherein:
one of R³ or R⁶ is independently selected from the group consisting of: F, Cl, Br, and I; and
the other of R³ or R⁶ is **Z**-C≡C.

15. A compound according to Claim 1 of Formula C 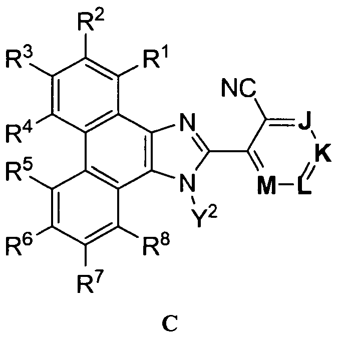 or a pharmaceutically acceptable salt thereof, wherein:
Y² is selected from the group consisting of: (1) C₁₋₆alkyl; (2) PO₄-C₁₋₄alkyl-; (3) C₁₋₄alkyl-C(O)-O-CH₂-, wherein the C₁₋₄alkyl portion is optionally substituted with R³³-O-C(O)-; and (4) C₁₋₄alkyl-O-C(O)-; and
R³³ is selected from the group consisting of: (1) H; (2) C₁₋₄alkyl, (3) C₃₋₆cycloalkyl; (4) phenyl; (5) benzyl; and (6) pyridyl; said C₁₋₄alkyl, C₃₋₆cycloalkyl, phenyl, benzyl and pyridyl may each be optionally substituted with 1 to 3 substituents independently selected from the group consisting of: OH, F, Cl, Br and I.

16. A compound according to Claim 1 selected from the following table: 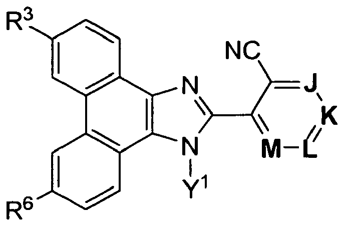
| **Ex** | **R³/R⁶** | **R⁶/R³** | **J** | **K** | **L** | **M** | **Y¹** |
|---|---|---|---|---|---|---|---|
| 1 | Cl | Br | CH | CH | CH | CF | H |
| 2 | H | H | CH | CH | CH | CH | H |
| 3 | CN | 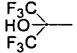 | CH | CH | CH | CF | H |
| 4 | Cl | 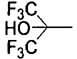 | CH | CH | CH | CF | H |
| 5 | Cl | H | CH | CH | CH | CF | H |
| 6 | CN | H | CH | CH | CH | CF | H |
| 7 | CN | 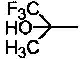 | CH | CH | CH | CF | H |
| 8 | Cl | 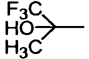 | CH | CH | CH | CF | H |
| 9 | Br | Br | CH | CH | CH | CF | H |
| 10 | H | H | CH | CH | CH | CCl | H |
| 11 | H | H | CH | CH | CH | CCN | H |
| 12 | 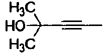 | Br | CH | CH | CH | CF | H |
| 13 | 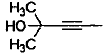 | 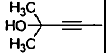 | CH | CH | CH | CF | H |
| 14 | 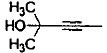 | Cl | CH | CH | CH | CF | H |
| 15 | 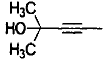 | I | CH | CH | CH | CF | H |
| 16 | H | H | CH | CH | CH | CBr | H |
| 17 | H | H | CH | CH | CH | CF | H |
| 18 | H | H | CH | N | CH | CCl | H |
| 19 | 3-pyridyl | 3-pyridyl | CH | CH | CH | CF | H |
| 20 | Cl | 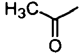 | CH | CH | CH | CF | H |
| 21 | Cl | 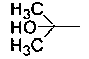 | CH | CH | CH | CF | H |
| 22 | 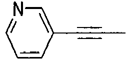 | Br | CH | CH | CH | CF | H |
| 23 | Cl | H | CH | N | CH | CCN | H |
| 24 | H | H | CH | N | CH | CCN | H |
| 25 | Cl | H | CH | CH | CH | CCN | H |
| 26 | H | H | CH | N | CH | CH | H |
| 27 | 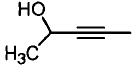 | Br | CH | CH | CH | CF | H |
| 28 | 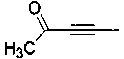 | Br | CH | CH | CH | CF | H |
| 29 | 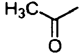 | 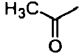 | CH | CH | CH | CF | H |
| 30 | 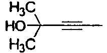 | 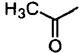 | CH | CH | CH | CF | H |
| 31 | H | H | N | CH | CH | N | H |
| 32 | H | H | N | CH | CH | CH | H |
| 33 | Br | 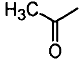 | CH | CH | CH | CF | H |
| 34 | I | I | CH | CH | CH | CF | H |
| 35 | Br | 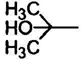 | CH | CH | CH | CF | H |
| 36 | Br | Cl | CH | CH | CH | CCN | H |
| 37 | Cl | 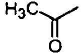 | CH | CH | CH | CBr | H |
| 38 | Cl | 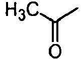 | CH | CH | CH | CCN | H |
| 39 | I | I | CH | CH | CH | CCN | H |
| 40 | 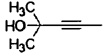 | Cl | CH | CH | CH | CCN | H |
| 41 | Cl | 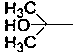 | CH | CH | CH | CCN | H |
| 42 | 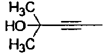 | I | CH | CH | CH | CCN | H |
| 43 | 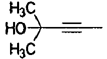 | 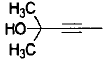 | CH | CH | CH | CCN | H |
| 44 | H | H | CH | CH | CH | CCN | CO₂Et |
| 45 | H | H | CH | CH | CH | CCN | 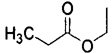 |
| 46 | 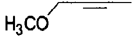 | Cl | CH | CH | CH | CCN | H |
| 47 | 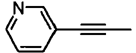 | Cl | CH | CH | CH | CCN | H |
| 48 | 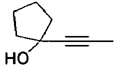 | Cl | CH | CH | CH | CCN | H |
| 49 | 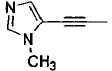 | Cl | CH | CH | CH | CCN | H |
| 50 | 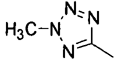 | Cl | CH | CH | CH | CCN | H |
| 51 | Cl | 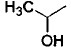 | CH | CH | CH | CCN | H |
| 52 | 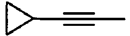 | Cl | CH | CH | CH | CCN | H |
| 53 | 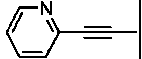 | Cl | CH | CH | CH | CCN | H |
| 54 | 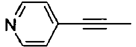 | Cl | CH | CH | CH | CCN | H |
| 55 | 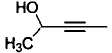 | Cl | CH | CH | CH | CCN | H |
| 56 | 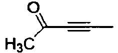 | Cl | CH | CH | CH | CCN | H |
| 57 | 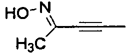 | Cl | CH | CH | CH | CCN | H |
| 58 | 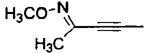 | Cl | CH | CH | CH | CCN | H |
| 59 | H | H | CH | CH | CH | CCN | 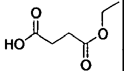 |
| 60 | H | H | CH | CH | CH | CCN | H₂PO₄CH 2 |
| 61 | 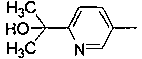 | Cl | CH | CH | CH | CCN | H |
| 62 | Cl | SO₂CH₃ | CH | CH | CH | CCN | H |
| 63 | Cl | 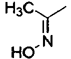 | CH | CH | CH | CCN | H |
| 64 | Br | H | CH | CH | CH | CCN | H |
| 65 | Cl | 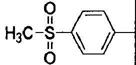 | CH | CH | CH | CCN | H |
| 66 | I | H | CH | CH | CH | CCN | H |
| 67 | CN | H | CH | CH | CH | CCN | H |
| 68 | cyclopropyl | Cl | CH | CH | CH | CCN | H |
| 69 | 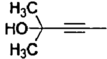 | 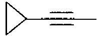 | CH | CH | CH | CCN | H |
| 70 | Cl | F | CH | CH | CH | CCN | H |
| 71 | Cl | 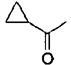 | CH | CH | CH | CCN | H |
| 72 | Cl | 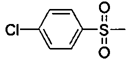 | CH | CH | CH | CCN | H |
| 73 | vinyl | H | CH | CH | CH | CCN | H |
| 74 | ethyl | H | CH | CH | CH | CCN | H |
| 75 | cyclopropyl | H | CH | CH | CH | CCN | H |
| 76 | Cl | 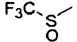 | CH | CH | CH | CBr | H |
| 77 | Cl | 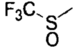 | CH | CH | CH | CCN | H |
| 78 | Cl | SO₂CF₃ | CH | CH | CH | CCN | H |
| 79 | 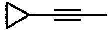 | H | CH | CH | CH | CCN | H |
| 80 | Cl | 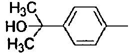 | CH | CH | CH | CCN | H |
| 81 | 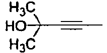 | Br | CH | CH | CH | CCN | H |
| 82 | Cl | 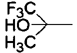 | CH | CH | CH | CCN | H |
| 83 | 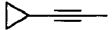 | 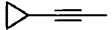 | CH | CH | CH | CCN | H |
| 84 | 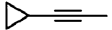 | 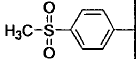 | CH | CH | CH | CCN | H |
| 85 | 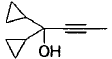 | Cl | CH | CH | CH | CCN | H |
| 86 | 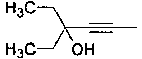 | Cl | CH | CH | CH | CCN | H |
| 87 | Br | 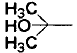 | CH | CH | CH | CCN | H |
| 88 | 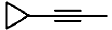 | 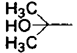 | CH | CH | CH | CCN | H |
| 89 | 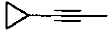 | CN | CH | CH | CH | CCN | H |
| 90 | 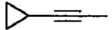 | CO₂CH₃ | CH | CH | CH | CCN | H |
| 91 | 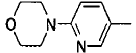 | Cl | CH | CH | CH | CCN | H |
| 92 | Cl | CN | CH | CH | CH | CCN | H |
| 93 | Cl | 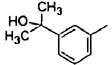 | CH | CH | CH | CCN | H |
| 94 | Br | 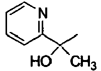 | CH | CH | CH | CCN | H |
| 95 | 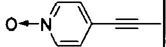 | Cl | CH | CH | CH | CCN | H |
| 96 | 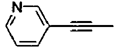 | 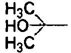 | CH | CH | CH | CCN | H |
| 97 | 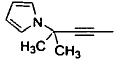 | Cl | CH | CH | CH | CCN | H |
| 98 | 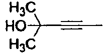 | Br | CH | CH | CH | CCl | H |
| 99 | 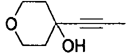 | Br | CH | CH | CH | CCl | H |
| 100 | Cl | CO₂*i*-Pr | CH | CH | CH | CCN | H |
| 101 | Cl | 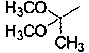 | CH | CH | CH | CF | H |
| 102 | 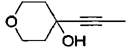 | Br | CH | CH | CH | CCN | H |
| 103 | 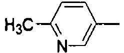 | Cl | CH | CH | CH | CCN | H |
| 104 | Br | 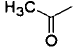 | CH | CH | CH | CCN | H |
| 105 | 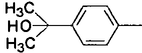 | Cl | CH | CH | CH | CCl | H |
| 106 | Br | 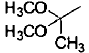 | CH | CH | CH | CCN | H |
| 107 | 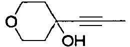 | Cl | CH | CH | CH | CCl | H |
| 108 | 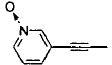 | Cl | CH | CH | CH | CCN | H |
| 109 | 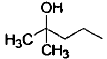 | Br | CH | CH | CH | CCN | H |
| 110 | 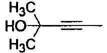 | Cl | CH | CH | CH | CCl | H |
| 111 | 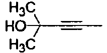 | 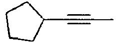 | CH | CH | CH | CCN | H |
| 112 | 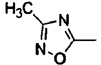 | Br | CH | CH | CH | CCN | H |
| 113 | 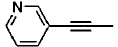 | 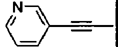 | CH | CH | CH | CCN | H |
| 114 | Et | 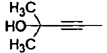 | CH | CH | CH | CCN | H |
| 115 | 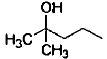 | 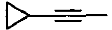 | CH | CH | CH | CCN | H |
| 116 | Br | 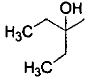 | CH | CH | CH | CCN | H |
| 117 | 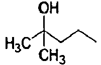 | Cl | CH | CH | CH | CCN | H |
| 118 | Br | CH3 | CH | CH | CH | CCN | H |
| 119 | 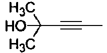 | CH3 | CH | CH | CH | CCN | H |
| 120 | 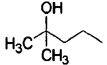 | CH3 | CH | CH | CH | CCN | H |
| 121 | 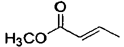 | Cl | CH | CH | CH | CCN | H |
| 122 | 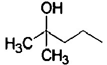 | H | CH | CH | CH | CCN | H |
| 123 | 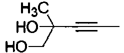 | Cl | CH | CH | CH | CCN | H |
or a pharmaceutically acceptable salt of any of the above.

17. A pharmaceutical composition comprising a compound according to any previous claim in combination with a pharmaceutically acceptable carrier.

18. A compound according to any of claims 1-16 for use in treating a microsomal prostaglandin E synthase-1 mediated disease or condition in a human patient in need of such treatment.

19. The compound for use according to Claim 18 wherein the disease or condition is selected from the group consisting of: acute or chronic pain, osteoarthritis, rheumatoid arthritis, bursitis, ankylosing spondylitis and primary dysmenorrhea.

## Patentansprüche

1. Eine Verbindung, dargestellt durch Formel I 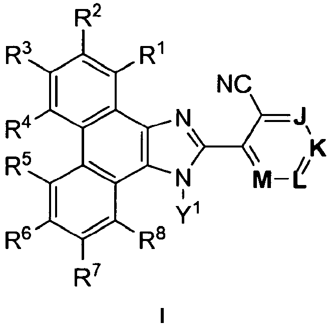 oder ein pharmazeutisch annehmbares Salz der Verbindung, wobei:
Y¹ H ist oder ausgewählt ist aus der Gruppe, bestehend aus: (1) C₁₋₆-Alkyl, (2) PO₄-C₁₋₄-Alkyl-, (3) C₁₋₄-Alkyl-C(O)-O-CH₂-, wobei der C₁₋₄-Alkylteil gegebenenfalls substituiert ist mit R³³-O-C(O)-, und (4) C₁₋₄-Alkyl-O-C(O)-,
R³³ ausgewählt ist aus der Gruppe, bestehend aus: (1) H, (2) C₁₋₄-Alkyl, (3) C₃₋₆-Cycloalkyl, (4) Phenyl, (5) Benzyl und (6) Pyridyl, wobei das C₁₋₄-Alkyl, C₃₋₆-Cycloalkyl, Phenyl, Benzyl und
Pyridyl jeweils gegebenenfalls substituiert sein können mit 1 bis 3 Substituenten, unabhängig ausgewählt aus der Gruppe, bestehend aus: OH, F, Cl, Br und I,
J ausgewählt ist aus der Gruppe, bestehend aus -C(X²)- und -N-,
**K** ausgewählt ist aus der Gruppe, bestehend aus -C(X³)- und -N-,
L ausgewählt ist aus der Gruppe, bestehend aus -C(X⁴)- und -N- und
**M** ausgewählt ist aus der Gruppe, bestehend aus -C(X⁵)- und -N-,
mit der Maßgabe, dass wenigstens eines von **J, K, L,** oder **M** anders als -N- ist,
X², X³, X⁴ und X⁵ jeweils unabhängig ausgewählt sind aus der Gruppe, bestehend aus: (1) H, (2) -CN, (3) F, (4) Cl, (5) Br, (6) I, (7) -OH, (8) -N₃, (9) C₁₋₆-Alkyl, C₂₋₆-Alkenyl oder C₂₋₆-Alkinyl, wobei eines oder mehrere der Wasserstoffatome, die an das C₁₋₆-Alkyl, C₂₋₆-Alkenyl oder C₂₋₆-Alkinyl gebunden sind, durch ein Fluoratom ersetzt sein können, und wobei das C₁₋₆-Alkyl, C₂₋₆-Alkenyl oder C₂₋₆-Alkinyl gegebenenfalls substituiert sein kann mit einer Hydroxygruppe, (10) C₁₋₄-Alkoxy, (11) NR⁹R¹⁰-C(O)-C₁₋₄-Alkyl-O-, (12) C₁₋₄-Alkyl-S(O)ₖ, (13) -NO₂, (14) C₃₋₆-Cycloalkyl, (15) C₃₋₆-Cycloalkoxy, (16) Phenyl, (17) Carboxy und (18) C₁₋₄-Alkyl-O-C(O)-,
R¹, R², R³, R⁴, R⁵, R⁶, R⁷ und R⁸ unabhängig ausgewählt sind aus der Gruppe, bestehend aus: (1) H, (2) F, (3) Cl, (4) Br, (5) I, (6) -CN, (7) C₁₋₆-Alkyl oder C₂₋₆-Alkenyl, wobei eines oder mehrere der Wasserstoffatome, die an das C₁₋₆-Alkyl oder C₂₋₆-Alkenyl gebunden sind, durch ein Fluoratom ersetzt sein können, und wobei das C₁₋₆-Alkyl oder C₂₋₆-Alkenyl gegebenenfalls substituiert sein kann mit einem bis drei Substituenten, unabhängig ausgewählt aus der Gruppe, bestehend aus: -OH, Methoxy, R¹¹-O-C(O)-, Cyclopropyl, Pyridyl und Phenyl, (8) C₃₋₆-Cycloalkyl, (9) R¹²-O-, (10) R¹³-S(O)ₖ-, (11) R¹⁴-S(O)ₖ-N(R¹⁵)-, (12) R¹⁶-C(O)-, (13) R¹⁷-N(R¹⁸)-, (14) R¹⁹N(R²⁰)-C(O)-, (15) R²¹-N(R²²)-S(O)ₖ, (16) R²³-C(O)-N(R²⁴)-, (17) **Z**-C≡C, (18) -(CH₃)C=N-OH oder -(CH₃)C=N-OCH₃, und (19) Phenyl, Naphthyl, Pyridyl, Pyridazinyl, Pyrimidinyl, Pyrazinyl, Pyrrolyl, Pyrazolyl, Imidazolyl, Triazolyl, Tetrazolyl, Oxazolyl, Isoxazolyl, Oxadiazolyl, Thienyl oder Furyl, wobei jedes gegebenenfalls substituiert ist mit einem Substituenten, unabhängig ausgewählt aus der Gruppe, bestehend aus: F, Cl, Br, I, C₁₋₄-Alkyl, Phenyl, Methylsulfonyl, Methylsulfonylamino, R²⁵-O-C(O)- und R²⁶-N(R²⁷)-, wobei das C₁₋₄-Alkyl gegebenenfalls substituiert ist mit 1 bis 3 Gruppen, unabhängig ausgewählt aus Halogen und Hydroxy,
jedes **Z** unabhängig ausgewählt ist aus der Gruppe, bestehend aus: (1) H, (2) C₁₋₆-Alkyl, wobei eines oder mehrere der Wasserstoffatome, die an das C₁₋₆-Alkyl gebunden sind, durch ein Fluoratom ersetzt sein können, und wobei C₁₋₆-Alkyl gegebenenfalls substituiert ist mit einem bis drei Substituenten, unabhängig ausgewählt aus: Hydroxy, Methoxy, Cyclopropyl, Phenyl, Pyridyl, Pyrrolyl, R²⁸-N(R²⁹)- und R³⁰-O-C(O)-, (3) -(CH₃)C=N-OH oder -(CH₃)C=N-OCH₃, (4) R³¹-C(O)-, (5) Phenyl, (6) Pyridyl oder dem N-Oxid davon, (7) C₃₋₆-Cycloalkyl, gegebenenfalls substituiert mit Hydroxy, (8) Tetrahydropyranyl, gegebenenfalls substituiert mit Hydroxy, und (9) einem fünfgliedrigen aromatischen Heterocyclus, der 1 bis 3 Atome, unabhängig ausgewählt aus O, N und S, enthält und gegebenenfalls mit Methyl substituiert ist,
jedes R⁹, R¹⁰, R¹⁵, R²⁴ und R³² unabhängig ausgewählt ist aus der Gruppe, bestehend aus: (1) H und (2) C₁₋₄-Alkyl,
jedes R¹¹, R¹², R¹³, R¹⁴, R¹⁶, R²³, R²⁵, R³⁰ und R³¹ unabhängig ausgewählt ist aus der Gruppe, bestehend aus: (1) H, (2) C₁₋₄-Alkyl, (3) C₃₋₆-Cycloalkyl, (4) Phenyl, (5) Benzyl und (6) Pyridyl, wobei das C₁₋₄-Alkyl, C₃₋₆-Cycloalkyl, Phenyl, Benzyl und Pyridyl gegebenenfalls substituiert sein kann mit 1 bis 3 Substituenten, unabhängig ausgewählt aus der Gruppe, bestehend aus: OH, F, Cl, Br und I,
jedes R¹⁷, R¹⁸, R¹⁹, R²⁰, R²¹, R²², R²⁶, R²⁷, R²⁸ und R²⁹ unabhängig ausgewählt ist aus der Gruppe, bestehend aus: (1) H, (2) C₁₋₆-Alkyl, (3) C₁₋₆-Alkoxy, (4) OH und (5) Benzyl oder 1-Phenylethyl, und R¹⁷ und R¹⁸, R¹⁹ und R²⁰, R²¹ und R²², R²⁶ und R²⁷, und R²⁸ und R²⁹ zusammen mit dem Stickstoffatom, an das sie gebunden sind, verbunden sein können unter Bildung eines monocyclichen Rings aus 5 oder 6 Kohlenstoffatomen, der gegebenenfalls ein oder zwei Atome, unabhängig ausgewählt aus -O-, -S(O)ₖ- und -N(R³²)-, enthält, und
jedes k unabhängig 0, 1 oder 2 ist.

2. Eine Verbindung gemäß Anspruch 1 gemäß Formel A 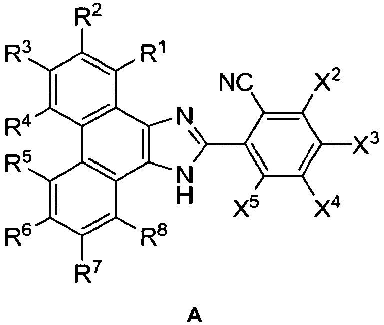 oder ein pharmazeutisch annehmbares Salz dieser Verbindung.

3. Die Verbindung gemäß Anspruch 2, wobei:
X², X³, X⁴ und X⁵ unabhängig ausgewählt sind aus der Gruppe, bestehend aus: (1) H, (2) -CN, (3) F, (4) Cl, (5) Br und (6) I.

4. Die Verbindung gemäß Anspruch 2, wobei X², X³ und X⁴ H sind und X⁵ anders als H ist.

5. Die Verbindung gemäß Anspruch 4, wobei X⁵ -CN ist.

6. Die Verbindung gemäß Anspruch 2, wobei wenigstens eines von R¹ oder R⁸ anders als H ist.

7. Die Verbindung gemäß Anspruch 2, wobei wenigstens eines von R² oder R⁷ anders als H ist.

8. Die Verbindung gemäß Anspruch 2, wobei wenigstens eines von R⁴ oder R⁵ anders als H ist.

9. Die Verbindung gemäß Anspruch 2, wobei:
wenigstens eines von R³ oder R⁶ anders als H ist, und
R¹, R², R⁴, R⁵, R⁷ und R⁸ H sind.

10. Die Verbindung gemäß Anspruch 9, wobei R³ und R⁶ beide anders als H sind.

11. Die Verbindung gemäß Anspruch 10, wobei:
eines von R³ oder R⁶ unabhängig ausgewählt ist aus der Gruppe, bestehend aus: F, Cl, Br und I, und
das andere von R³ oder R⁶ **Z**-C≡C ist.

12. Die Verbindung gemäß Anspruch 9, wobei: R³ und R⁶ unabhängig ausgewählt sind aus der Gruppe, bestehend aus: Wasserstoff, Fluor, Chlor, Brom, Iod, Cyano, Methyl, Ethyl, Vinyl, Cyclopropyl, -CO₂*i*Pr, -CO₂CH₃, -SO₂CF₃, 3-Pyridyl, Acetyl, 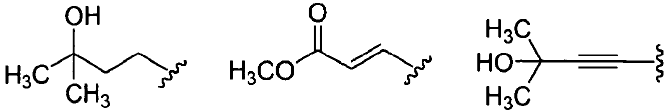 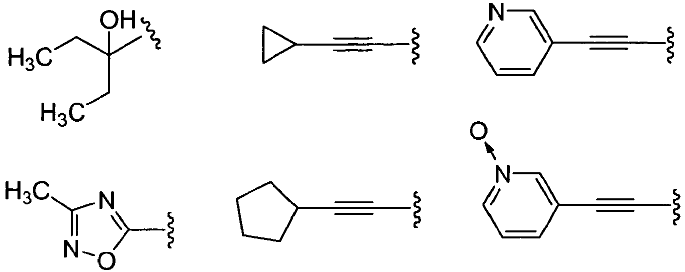 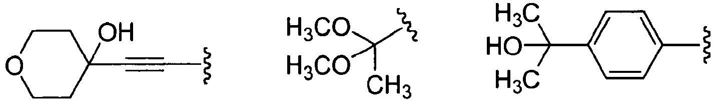 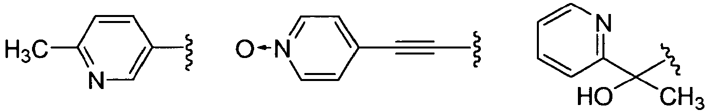 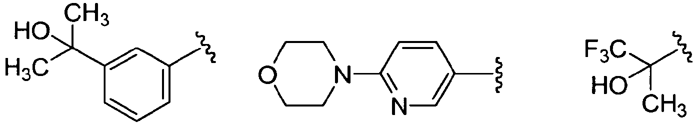 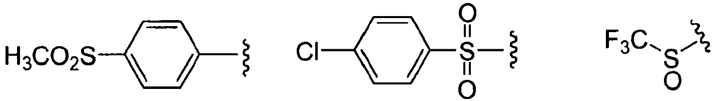 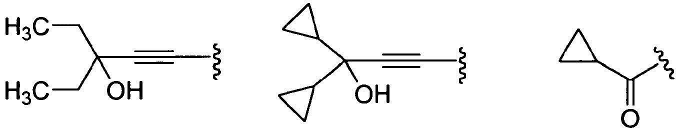 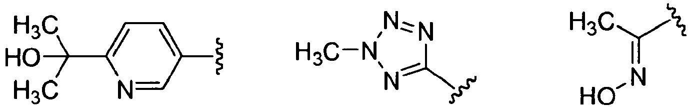 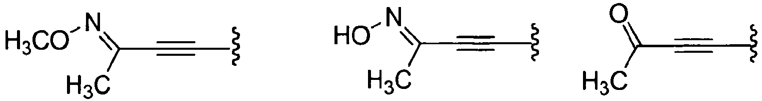 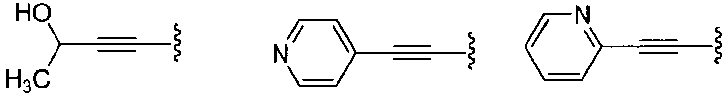 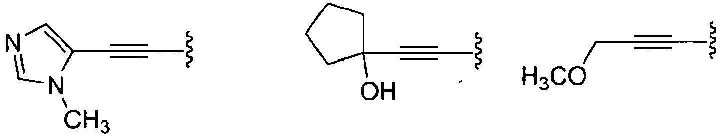 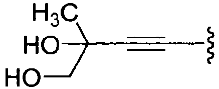 mit der Maßgabe, dass wenigstens eines von R³ oder R⁶ anders als H ist.

13. Die Verbindung gemäß Anspruch 1 gemäß Formel **B**: 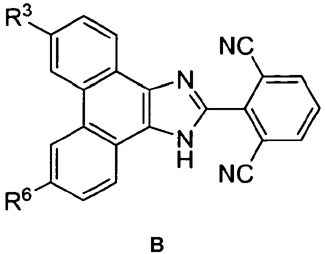 oder ein pharmazeutisch annehmbares Salz dieser Verbindung.

14. Die Verbindung gemäß Anspruch 13, wobei:
eines von R³ oder R⁶ unabhängig ausgewählt ist aus der Gruppe, bestehend aus: F, Cl, Br und I, und
das andere von R³ oder R⁶ **Z**-C≡C ist.

15. Eine Verbindung gemäß Anspruch 1 oder Formel C 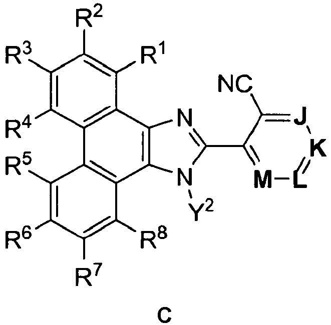 oder ein pharmazeutisch annehmbares Salz davon, wobei:
Y² ausgewählt ist aus der Gruppe, bestehend aus: (1) C₁₋₆-Alkyl, (2) PO₄-C₁₋₄-Alkyl-, (3) C₁₋₄-Alkyl-C(O)-O-CH₂-, wobei der C₁₋₄-Alkylteil gegebenenfalls substituiert ist mit R³³-O-C(O)-, und (4) C₁₋₄-Alkyl-O-C(O)-, und
R³³ ausgewählt ist aus der Gruppe, bestehend aus: (1) H, (2) C₁₋₄-Alkyl, (3) C₃₋₆-Cycloalkyl, (4) Phenyl, (5) Benzyl und (6) Pyridyl, wobei das C₁₋₄-Alkyl, C₃₋₆-Cycloalkyl, Phenyl, Benzyl und Pyridyl jeweils gegebenenfalls substituiert sein können mit 1 bis 3 Substituenten, unabhängig ausgewählt aus der Gruppe, bestehend aus: OH, F, Cl, Br und I.

16. Eine Verbindung gemäß Anspruch 1, ausgewählt aus der folgenden Tabelle: 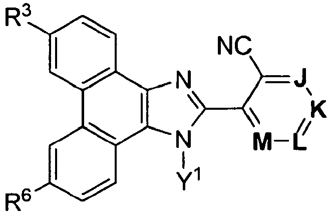
| **Bsp.** | **R³/R⁶** | **R⁶/R³** | **J** | **K** | **L** | **M** | **Y¹** |
|---|---|---|---|---|---|---|---|
| 1 | Cl | Br | CH | CH | CH | CF | H |
| 2 | H | H | CH | CH | CH | CH | H |
| 3 | CN | 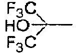 | CH | CH | CH | CF | H |
| 4 | Cl | 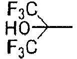 | CH | CH | CH | CF | H |
| 5 | Cl | H | CH | CH | CH | CF | H |
| 6 | CN | H | CH | CH | CH | CF | H |
| 7 | CN | 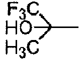 | CH | CH | CH | CF | H |
| 8 | Cl | 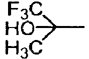 | CH | CH | CH | CF | H |
| 9 | Br | Br | CH | CH | CH | CF | H |
| 10 | H | H | CH | CH | CH | CCl | H |
| 11 | H | H | CH | CH | CH | CCN | H |
| 12 | 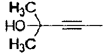 | Br | CH | CH | CH | CF | H |
| 13 | 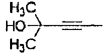 | 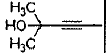 | CH | CH | CH | CF | H |
| 14 | 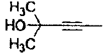 | Cl | CH | CH | CH | CF | H |
| 15 | 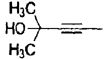 | I | CH | CH | CH | CF | H |
| 16 | H | H | CH | CH | CH | CBr | H |
| 17 | H | H | CH | CH | CH | CF | H |
| 18 | H | H | CH | N | CH | CCl | H |
| 19 | 3-Pyridyl | 3-Pyridyl | CH | CH | CH | CF | H |
| 20 | Cl | 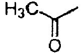 | CH | CH | CH | CF | H |
| 21 | Cl | 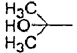 | CH | CH | CH | CF | H |
| 22 | 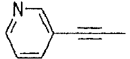 | Br | CH | CH | CH | CF | H |
| 23 | Cl | H | CH | N | CH | CCN | H |
| 24 | H | H | CH | N | CH | CCN | H |
| 25 | Cl | H | CH | CH | CH | CCN | H |
| 26 | H | H | CH | N | CH | CH | H |
| 27 | 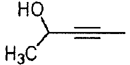 | Br | CH | CH | CH | CF | H |
| 28 | 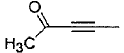 | Br | CH | CH | CH | CF | H |
| 29 | 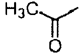 | 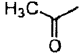 | CH | CH | CH | CF | H |
| 30 | 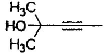 | 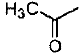 | CH | CH | CH | CF | H |
| 31 | H | H | N | CH | CH | N | H |
| 32 | H | H | N | CH | CH | CH | H |
| 33 | Br | 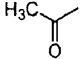 | CH | CH | CH | CF | H |
| 34 | I | I | CH | CH | CH | CF | H |
| 35 | Br | 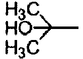 | CH | CH | CH | CF | H |
| 36 | Br | Cl | CH | CH | CH | CCN | H |
| 37 | Cl | 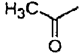 | CH | CH | CH | CBr | H |
| 38 | Cl | 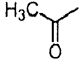 | CH | CH | CH | CCN | H |
| 39 | I | I | CH | CH | CH | CCN | H |
| 40 | 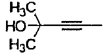 | Cl | CH | CH | CH | CCN | H |
| 41 | Cl | 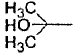 | CH | CH | CH | CCN | H |
| 42 | 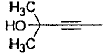 | I | CH | CH | CH | CCN | H |
| 43 | 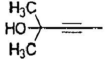 | 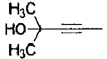 | CH | CH | CH | CCN | H |
| 44 | H | H | CH | CH | CH | CCN | CO₂Et |
| 45 | H | H | CH | CH | CH | CCN | 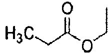 |
| 46 | 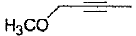 | Cl | CH | CH | CH | CCN | H |
| 47 | 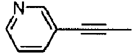 | Cl | CH | CH | CH | CCN | H |
| 48 | 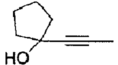 | Cl | CH | CH | CH | CCN | H |
| 49 | 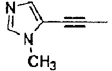 | Cl | CH | CH | CH | CCN | H |
| 50 | 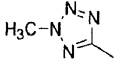 | Cl | CH | CH | CH | CCN | H |
| 51 | Cl | 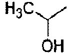 | CH | CH | CH | CCN | H |
| 52 | 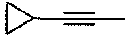 | Cl | CH | CH | CH | CCN | H |
| 53 | 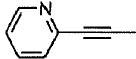 | Cl | CH | CH | CH | CCN | H |
| 54 | 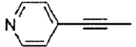 | Cl | CH | CH | CH | CCN | H |
| 55 | 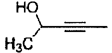 | Cl | CH | CH | CH | CCN | H |
| 56 | 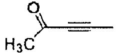 | Cl | CH | CH | CH | CCN | H |
| 57 | 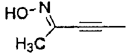 | Cl | CH | CH | CH | CCN | H |
| 58 | 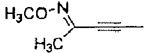 | Cl | CH | CH | CH | CCN | H |
| 59 | H | H | CH | CH | CH | CCN | 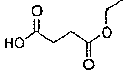 |
| 60 | H | H | CH | CH | CH | CCN | H₂PO₄CH 2 |
| 61 | 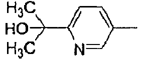 | Cl | CH | CH | CH | CCN | H |
| 62 | Cl | SO₂CH₃ | CH | CH | CH | CCN | H |
| 63 | Cl | 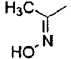 | CH | CH | CH | CCN | H |
| 64 | Br | H | CH | CH | CH | CCN | H |
| 65 | Cl | 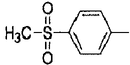 | CH | CH | CH | CCN | H |
| 66 | I | H | CH | CH | CH | CCN | H |
| 67 | CN | H | CH | CH | CH | CCN | H |
| 68 | Cyclopropyl | Cl | CH | CH | CH | CCN | H |
| 69 | 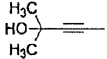 | 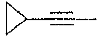 | CH | CH | CH | CCN | H |
| 70 | Cl | F | CH | CH | CH | CCN | H |
| 71 | Cl | 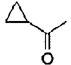 | CH | CH | CH | CCN | H |
| 72 | Cl | 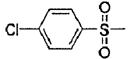 | CH | CH | CH | CCN | H |
| 73 | Vinyl | H | CH | CH | CH | CCN | H |
| 74 | Ethyl | H | CH | CH | CH | CCN | H |
| 75 | Cyclopropyl | H | CH | CH | CH | CCN | H |
| 76 | Cl | 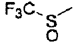 | CH | CH | CH | CBr | H |
| 77 | Cl | 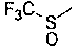 | CH | CH | CH | CCN | H |
| 78 | Cl | SO₂CF₃ | CH | CH | CH | CCN | H |
| 79 | 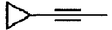 | H | CH | CH | CH | CCN | H |
| 80 | Cl | 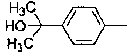 | CH | CH | CH | CCN | H |
| 81 | 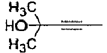 | Br | CH | CH | CH | CCN | H |
| 82 | Cl | 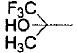 | CH | CH | CH | CCN | H |
| 83 | 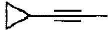 | 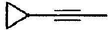 | CH | CH | CH | CCN | H |
| 84 | 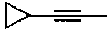 | 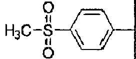 | CH | CH | CH | CCN | H |
| 85 | 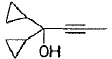 | Cl | CH | CH | CH | CCN | H |
| 86 | 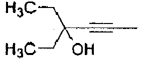 | Cl | CH | CH | CH | CCN | H |
| 87 | Br | 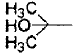 | CH | CH | CH | CCN | H |
| 88 | 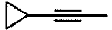 | 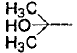 | CH | CH | CH | CCN | H |
| 89 | 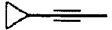 | CN | CH | CH | CH | CCN | H |
| 90 | 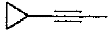 | CO₂CH₃ | CH | CH | CH | CCN | H |
| 91 | 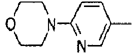 | Cl | CH | CH | CH | CCN | H |
| 92 | Cl | CN | CH | CH | CH | CCN | H |
| 93 | Cl | 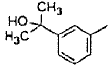 | CH | CH | CH | CCN | H |
| 94 | Br | 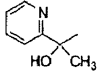 | CH | CH | CH | CCN | H |
| 95 | 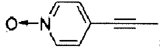 | Cl | CH | CH | CH | CCN | H |
| 96 | 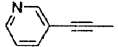 | 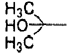 | CH | CH | CH | CCN | H |
| 97 | 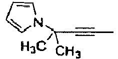 | Cl | CH | CH | CH | CCN | H |
| 98 | 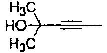 | Br | CH | CH | CH | CCl | H |
| 99 | 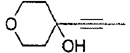 | Br | CH | CH | CH | CCl | H |
| 100 | Cl | CO₂*i*-Pr | CH | CH | CH | CCN | H |
| 101 | Cl | 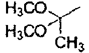 | CH | CH | CH | CF | H |
| 102 | 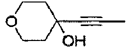 | Br | CH | CH | CH | CCN | H |
| 103 | 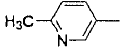 | Cl | CH | CH | CH | CCN | H |
| 104 | Br | 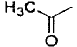 | CH | CH | CH | CCN | H |
| 105 | 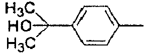 | Cl | CH | CH | CH | CCl | H |
| 106 | Br | 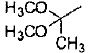 | CH | CH | CH | CCN | H |
| 107 | 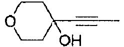 | Cl | CH | CH | CH | CCl | H |
| 108 | 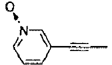 | Cl | CH | CH | CH | CCN | H |
| 109 | 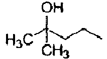 | Br | CH | CH | CH | CCN | H |
| 110 | 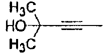 | Cl | CH | CH | CH | CCl | H |
| 111 | 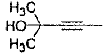 | 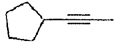 | CH | CH | CH | CCN | H |
| 112 | 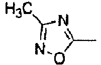 | Br | CH | CH | CH | CCN | H |
| 113 | 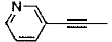 | 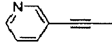 | CH | CH | CH | CCN | H |
| 114 | Et | 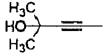 | CH | CH | CH | CCN | H |
| 115 | 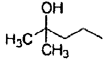 | 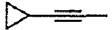 | CH | CH | CH | CCN | H |
| 116 | Br | 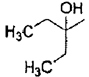 | CH | CH | CH | CCN | H |
| 117 | 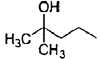 | Cl | CH | CH | CH | CCN | H |
| 118 | Br | CH3 | CH | CH | CH | CCN | H |
| 119 | 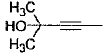 | CH3 | CH | CH | CH | CCN | H |
| 120 | 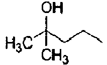 | CH3 | CH | CH | CH | CCN | H |
| 121 | 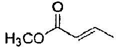 | Cl | CH | CH | CH | CCN | H |
| 122 | 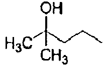 | H | CH | CH | CH | CCN | H |
| 123 | 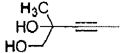 | Cl | CH | CH | CH | CCN | H |
oder ein pharmazeutisch annehmbares Salz von einer der obigen Verbindungen.

17. Eine pharmazeutische Zusammensetzung, umfassend eine Verbindung gemäß einem vorhergehenden Anspruch in Kombination mit einem pharmazeutisch annehmbaren Träger.

18. Eine Verbindung gemäß einem der Ansprüche 1-16 zur Verwendung bei der Behandlung einer durch mikrosomale Prostaglandin-E-Synthase 1 vermittelten Erkrankung oder eines durch mikrosomale Prostaglandin-E-Synthase 1 vermittelten Zustands bei einem menschlichen Patienten, der eine solche Behandlung benötigt.

19. Die Verbindung zur Verwendung nach Anspruch 18, wobei die Erkrankung oder der Zustand ausgewählt ist aus der Gruppe, bestehend aus: akuten oder chronischen Schmerzen, Arthrose, rheumatoider Arthritis, Bursitis, Spondylitis ankylosans und primärer Dysmenorrhö.

## Revendications

1. Composé représenté par la Formule I 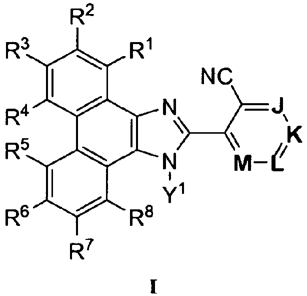 ou un sel pharmaceutiquement acceptable dudit composé, dans lequel:
Y¹ est H ou est sélectionné parmi le groupe consistant en: (1) alkyle C₁₋₆; (2) PO₄-alkyle C₁₋₄; (3) alkyle C₁₋₄-C(O)-O-CH₂-, où la portion alkyle C₁₋₄ est optionnellement substituée par R³³-O-C(O)- et (4) alkyle C₁₋₄-O-C(O)-;
R³³ est sélectionné parmi le groupe consistant en: (1) H; (2) alkyle C₁₋₄, (3) cycloalkyle C₃₋₆; (4) phényle; (5) benzyle et (6) pyridyle; lesdits alkyle C₁₋₄, cycloalkyle C₃₋₆, phényle, benzyle et pyridyle peuvent être chacun optionnellement substitués par 1 à 3 substituants sélectionnés indépendamment parmi le groupe consistant en: OH, F, Cl, Br et I;
J est sélectionné parmi le groupe consistant en -C(X²)- et -N-;
K est sélectionné parmi le groupe consistant en -C(X³)- et -N-;
L est sélectionné parmi le groupe consistant en -C(X⁴)- et -N-; et
M est sélectionné parmi le groupe consistant en -C(X⁵)- et -N-;
à condition qu'au moins l'un d'entre J, K, L ou M soit autre que -N-;
X² X³, X⁴ et X⁵ sont sélectionnés indépendamment parmi le groupe consistant en: (1) H; (2) -CN; (3) F; (4) Cl; (5) Br; (6) I; (7) -OH; (8) -N₃; (9) alkyle C₁₋₆, alcényle C₂₋₆ ou alcynyle C₂₋₆, où un ou plusieurs des atomes d'hydrogène attachés auxdits alkyle C₁₋₆, alcényle C₂-₆ ou alcynyle C₂₋₆, peuvent être remplacés par un atome de fluoro et lesdits alkyle C₁₋₆, alcényle C₂₋₆ ou alcynyle C₂₋₆ peuvent être optionnellement substitués par un groupe hydroxy; (10) alcoxy C₁₋₄; (11) NR⁹R¹⁰-C(O)-alkyle C₁₋₄-O-; (12) alkyle C₁₋₄-S(O)ₖ-; (13) -NO₂; (14) cycloalkyle C₃₋₆, (15) cycloalcoxy C₃₋₆; (16) phényle, (17) carboxy et (18) alkyle C₁₋₄-O-C(O)-;
R¹, R², R³, R⁴, R⁵, R⁶, R⁷ et R⁸ sont sélectionnés indépendamment parmi le groupe consistant en: (1) H; (2) F; (3) Cl; (4) Br; (5) I; (6) -CN; (7) alkyle C₁₋₆ ou alcényle C₂₋₆, où un ou plusieurs des atomes d'hydrogène attachés auxdits alkyle C₁₋₆ ou alcényle C₂₋₆ peuvent être remplacés par un atome de fluoro et où lesdits alkyle C₁₋₆ ou alcényle C₂₋₆ peuvent être optionnellement substitués par un à trois substituants sélectionnés indépendamment parmi le groupe consistant en: -OH, méthoxy, R¹¹-O-C(O)-, cyclopropyle, pyridyle et phényle; (8) cycloalkyle C₃₋₆; (9) R¹²-O-; (10) R¹³-S(O)ₖ-, (11) R¹⁴-S(O)ₖ-N(R¹⁵)-; (12) R¹⁶-C(O)-; (13) R¹⁷-N(R¹⁸)-; (14) R¹⁹-N(R²⁰)-C(O)-; (15) R²¹-N(R²²)-S(O)ₖ-; (16) R²³-C(O)-N(R²⁴)-; (17) Z-C≡C; (18) -(CH₃)C=N-OH ou - (CH₃)C=N-OCH₃ et (19) phényle, naphtyle, pyridyle, pyradazinyle, pyrimidinyle, pyrazinyle, pyrrolyle, pyrazolyle, imidazolyle, triazolyle, tétrazolyle, oxazolyle, isoxazolyle, oxadiazolyle, thiényle ou furyle, chacun optionnellement substitué par un substituant sélectionné indépendamment parmi le groupe consistant en: F, Cl, Br, I, alkyle C₁₋₄, phényle, méthylsulfonyle, méthylsulfonylamino, R²⁵-O-C(O)- et R²⁶-N(R²⁷)-, ledit alkyle C₁₋₄ optionnellement substitué par 1 à 3 groupes sélectionnés indépendamment parmi halo et hydroxy;
chaque Z est sélectionné indépendamment parmi le groupe consistant en: (1) H; (2) alkyle C₁₋₆, où un ou plusieurs des atomes d'hydrogène attachés audit alkyle C₁₋₆ peuvent être remplacés par un atome de fluoro et où l'alkyle C₁₋₆ peut être optionnellement substitué par un à trois substituants sélectionnés indépendamment parmi: hydroxy, méthoxy, cyclopropyle, phényle, pyridyle, pyrrolyle, R²⁸-N(R²⁹)- et R³⁰-OC(O)-; (3) -(CH₃)C=N-OH ou -(CH₃)C=N-OCH₃; (4) R³¹-C(O)-; (5) phényle; (6) pyridyle ou le N-oxyde de celui-ci; (7) cycloalkyle C₃₋₆, optionnellement substitué par hydroxy; (8) tétrahydropyranyle, optionnellement substitué par hydroxy et (9) un hétérocycle aromatique à cinq chaînons contenant 1 à 3 atomes sélectionnés indépendamment parmi O, N ou S et optionnellement substitué par un méthyle;
chaque R⁹, R¹⁰, R¹⁵, R²⁴ et R³² est sélectionné indépendamment parmi le groupe consistant en: (1) H et (2) alkyle C₁₋₄;
chaque R¹¹, R¹², R¹³, R¹⁴, R¹⁶R²³, R²⁵, R³⁰ et R³¹ est sélectionné indépendamment parmi le groupe consistant en: (1) H; (2) alkyle C₁₋₄, (3) cycloalkyle C₃₋₆; (4) phényle, (5) benzyle et (6) pyridyle; lesdits alkyle C₁₋₄, cycloalkyle C₃₋₆, phényle, benzyle et pyridyle peuvent être chacun optionnellement substitués par 1 à 3 substituants sélectionnés indépendamment parmi le groupe consistant en: OH, F, Cl, Br et I;
chaque R¹⁷, R¹⁸, R¹⁹, R²⁰, R²¹, R²², R²⁶, R²⁷, R²⁸ et R²⁹ est sélectionné indépendamment parmi le groupe consistant en: (1) H; (2) alkyle C₁₋₆; (3) alcoxy C₁₋₆; (4) OH et (5) benzyle ou 1-phényléthyle; et R¹⁷ et R¹⁸, R¹⁹et R²⁰, R²¹ et R²², R²⁶ et R²⁷, et R²⁸ et R²⁹, peuvent être joints ensemble avec l'atome d'azote auquel ils sont attachés pour former un cycle monocyclique de 5 ou 6 atomes de carbone, contenant optionnellement un ou deux atomes sélectionnés indépendamment parmi -O-, -S(O)ₖ- et -N(R³²)-; et
chaque k est indépendamment 0, 1 ou 2.

2. Composé selon la revendication 1 selon la Formule A 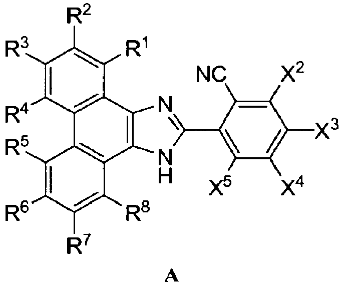 ou un sel pharmaceutiquement acceptable dudit composé.

3. Composé selon la revendication 2, dans lequel:
X², X³, X⁴ et X⁵ sont sélectionnés indépendamment parmi le groupe consistant en: (1) H; (2) -CN; (3) F; (4) Cl; (5) Br et (6) I.

4. Composé selon la revendication 2, dans lequel X², X³ et X⁴ sont H et X⁵ est autre que H.

5. Composé selon la revendication 4, dans lequel X⁵ est -CN.

6. Composé selon la revendication 2, dans lequel au moins l'un de R¹ ou de R⁸ est autre que H.

7. Composé selon la revendication 2, dans lequel au moins l'un de R² ou de R⁷ est autre que H.

8. Composé selon la revendication 2, dans lequel au moins l'un de R⁴ ou de R⁵ est autre que H.

9. Composé selon la revendication 2, dans lequel au moins l'un de R³ ou de R⁶ est autre que H et R¹, R², R⁴, R⁵, R⁷ et R⁸ sont H.

10. Composé selon la revendication 9, dans lequel R³ et R⁶ sont tous les deux H.

11. Composé selon la revendication 10, dans lequel l'un de R³ ou R⁶ est sélectionné indépendamment parmi le groupe consistant en: F, Cl, Br et I et l'autre de R³ ou R⁶ est Z-C≡C.

12. Composé selon la revendication 9, dans lequel: R³ et R⁶ sont sélectionnés indépendamment parmi le groupe consistant en: hydrogène, fluoro, chloro, bromo, iodo, cyano, méthyle, éthyle, vinyle, cyclopropyle, -CO₂*i*-Pr, -CO₂CH₃, SO₂CF₃, 3-pyridyle, acétyle, 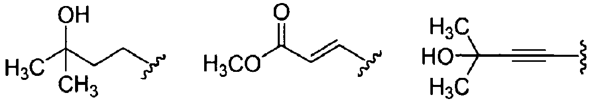 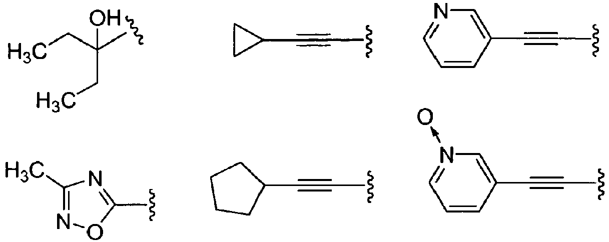 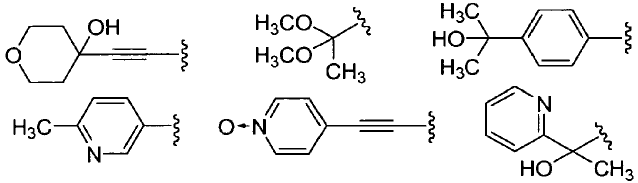 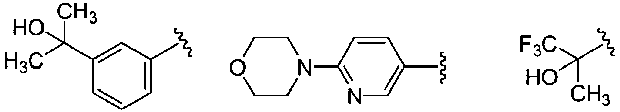 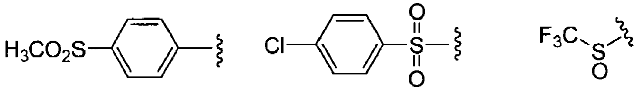 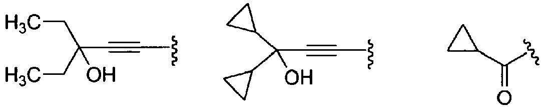 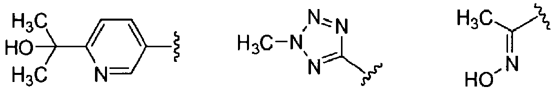 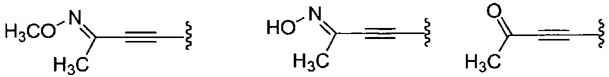 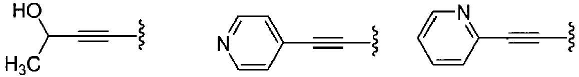 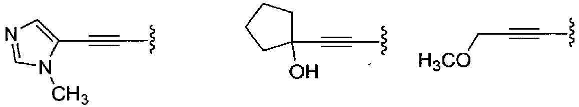 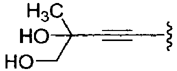 à condition qu'au moins l'un de R³ ou R⁶ soit autre que H.

13. Composé selon la revendication 1 selon la Formule B: 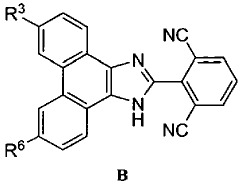 ou un sel pharmaceutiquement acceptable dudit composé.

14. Composé selon la revendication 13, dans lequel:
l'un de R³ ou R⁶ est sélectionné indépendamment parmi le groupe consistant en: F, Cl, Br et L et l'autre de R³ ou R⁶ est Z-C≡C.

15. Composé selon la revendication 1 de la Formule C: 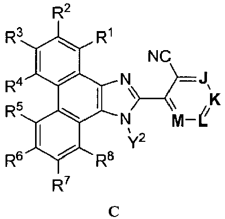 ou un sel pharmaceutiquement acceptable dudit composé, dans lequel:
Y² est sélectionné parmi le groupe consistant en: (1) alkyle C₁₋₆; (2) PO₄-alkyle C₁₋₄-; (3) alkyle C₁₋₄-C(O)-O-CH₂-, où la portion alkyle C₁₋₄ est optionnellement substituée par R³³-O-C(O)- et (4) alkyle C₁₋₄-O-C(O)-; et
R³³ est sélectionné parmi le groupe consistant en: (1) H; (2) alkyle C₁₋₄, (3) cycloalkyle C₃₋₆; (4) phényle; (5) benzyle et (6) pyridyle; lesdits alkyle C₁₋₄, cycloalkyle C₃₋₆, phényle, benzyle et pyridyle peuvent être chacun optionnellement substitués par 1 à 3 substituants sélectionnés indépendamment parmi le groupe consistant en: OH, F, Cl, Br et I.

16. Composé selon la revendication 1, sélectionné parmi la table suivante: 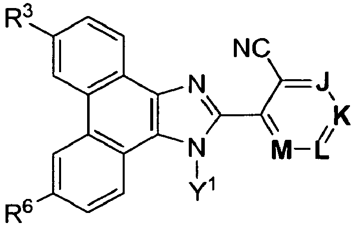
| **Ex** | **R³/R⁶** | **R⁶/R³** | **J** | **K** | **L** | **M** | **Y¹** |
|---|---|---|---|---|---|---|---|
| 1 | Cl | Br | CH | CH | CH | CF | H |
| 2 | H | H | CH | CH | CH | CH | H |
| 3 | CN | 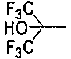 | CH | CH | CH | CF | H |
| 4 | Cl | 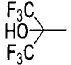 | CH | CH | CH | CF | H |
| 5 | Cl | H | CH | CH | CH | CF | H |
| 6 | CN | H | CH | CH | CH | CF | H |
| 7 | CN | 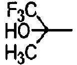 | CH | CH | CH | CF | H |
| 8 | Cl | 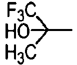 | CH | CH | CH | CF | H |
| 9 | Br | Br | CH | CH | CH | CF | H |
| 10 | H | H | CH | CH | CH | CCl | H |
| 11 | H | H | CH | CH | CH | CCN | H |
| 12 | 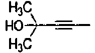 | Br | CH | CH | CH | CF | H |
| 13 | 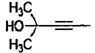 | 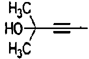 | CH | CH | CH | CF | H |
| 14 | 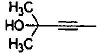 | Cl | CH | CH | CH | CF | H |
| 15 | 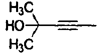 | I | CH | CH | CH | CF | H |
| 16 | H | H | CH | CH | CH | CBr | H |
| 17 | H | H | CH | CH | CH | CF | H |
| 18 | H | H | CH | N | CH | CCl | H |
| 19 | 3-pyridyl | 3-pyridyl | CH | CH | CH | CF | H |
| 20 | Cl | 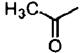 | CH | CH | CH | CF | H |
| 21 | Cl | 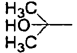 | CH | CH | CH | CF | H |
| 22 | 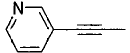 | Br | CH | CH | CH | CF | H |
| 23 | Cl | H | CH | N | CH | CCN | H |
| 24 | H | H | CH | N | CH | CCN | H |
| 25 | Cl | H | CH | CH | CH | CCN | H |
| 26 | H | H | CH | N | CH | CH | H |
| 27 | 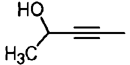 | Br | CH | CH | CH | CF | H |
| 28 | 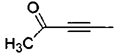 | Br | CH | CH | CH | CF | H |
| 29 | 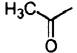 | 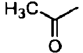 | CH | CH | CH | CF | H |
| 30 | 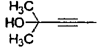 | 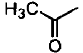 | CH | CH | CH | CF | H |
| 31 | H | H | N | CH | CH | N | H |
| 32 | H | H | N | CH | CH | CH | H |
| 33 | Br | 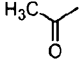 | CH | CH | CH | CF | H |
| 34 | I | I | CH | CH | CH | CF | H |
| 35 | Br | 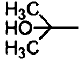 | CH | CH | CH | CF | H |
| 36 | Br | 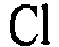 | CH | CH | CH | CCN | H |
| 37 | Cl | 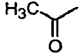 | CH | CH | CH | CBr | H |
| 38 | Cl | 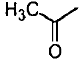 | CH | CH | CH | CCN | H |
| 39 | 1 | I | CH | CH | CH | CCN | H |
| 40 | 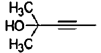 | Cl | CH | CH | CH | CCN | H |
| 41 | Cl | 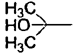 | CH | CH | CH | CCN | H |
| 42 | 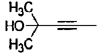 | I | CH | CH | CH | CCN | H |
| 43 | 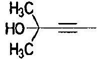 | 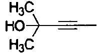 | CH | CH | CH | CCN | H |
| 44 | H | H | CH | CH | CH | CCN | CO₂Et |
| 45 | H | H | CH | CH | CH | CCN | 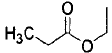 |
| 46 | 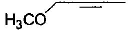 | Cl | CH | CH | CH | CCN | H |
| 47 | 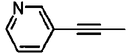 | Cl | CH | CH | CH | CCN | H |
| 48 | 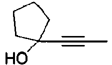 | Cl | CH | CH | CH | CCN | H |
| 49 | 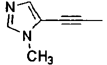 | Cl | CH | CH | CH | CCN | H |
| 50 | 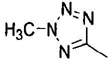 | Cl | CH | CH | CH | CCN | H |
| 51 | Cl | 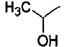 | CH | CH | CH | CCN | H |
| 52 | 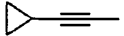 | Cl | CH | CH | CH | CCN | H |
| 53 | 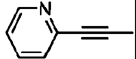 | Cl | CH | CH | CH | CCN | H |
| 54 | 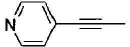 | Cl | CH | CH | CH | CCN | H |
| 55 | 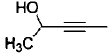 | Cl | CH | CH | CH | CCN | H |
| 56 | 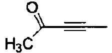 | Cl | CH | CH | CH | CCN | H |
| 57 | 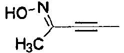 | Cl | CH | CH | CH | CCN | H |
| 58 | 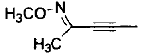 | Cl | CH | CH | CH | CCN | H |
| 59 | H | H | CH | CH | CH | CCN | 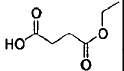 |
| 60 | H | H | CH | CH | CH | CCN | H₂PO₄CH 2 |
| 61 | 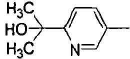 | Cl | CH | CH | CH | CCN | H |
| 62 | Cl | SO₂CH₃ | CH | CH | CH | CCN | H |
| 63 | Cl | 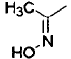 | CH | CH | CH | CCN | H |
| 64 | Br | H | CH | CH | CH | CCN | H |
| 65 | Cl | 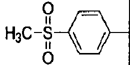 | CH | CH | CH | CCN | H |
| 66 | 1 | H | CH | CH | CH | CCN | H |
| 67 | CN | H | CH | CH | CH | CCN | H |
| 68 | cyclopropyl | Cl | CH | CH | CH | CCN | H |
| 69 | 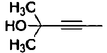 | 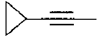 | CH | CH | CH | CCN | H |
| 70 | Cl | F | CH | CH | CH | CCN | H |
| 71 | Cl | 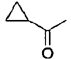 | CH | CH | CH | CCN | H |
| 72 | Cl | 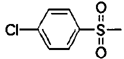 | CH | CH | CH | CCN | H |
| 73 | vinyl | H | CH | CH | CH | CCN | H |
| 74 | ethyl | H | CH | CH | CH | CCN | H |
| 75 | cyclopropyl | H | CH | CH | CH | CCN | H |
| 76 | Cl | 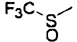 | CH | CH | CH | CBr | H |
| 77 | Cl | 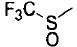 | CH | CH | CH | CCN | H |
| 78 | Cl | SO₂CF₃ | CH | CH | CH | CCN | H |
| 79 | 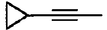 | H | CH | CH | CH | CCN | H |
| 80 | Cl | 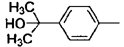 | CH | CH | CH | CCN | H |
| 81 | 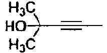 | Br | CH | CH | CH | CCN | H |
| 82 | l | 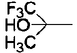 | CH | CH | CH | CCN | H |
| 83 | 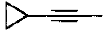 | 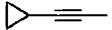 | CH | CH | CH | CCN | H |
| 84 | 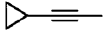 | 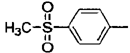 | CH | CH | CH | CCN | H |
| 85 | 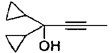 | Cl | CH | CH | CH | CCN | H |
| 86 | 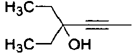 | Cl | CH | CH | CH | CCN | H |
| 87 | Br | 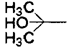 | CH | CH | CH | CCN | H |
| 88 | 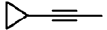 | 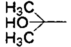 | CH | CH | CH | CCN | H |
| 89 | 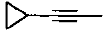 | CN | CH | CH | CH | CCN | H |
| 90 | 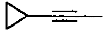 | CO₂CH₃ | CH | CH | CH | CCN | H |
| 91 | 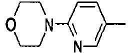 | Cl | CH | CH | CH | CCN | H |
| 92 | Cl | CN | CH | CH | CH | CCN | H |
| 93 | Cl | 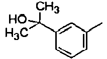 | CH | CH | CH | CCN | H |
| 94 | Br | 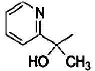 | CH | CH | CH | CCN | H |
| 95 | 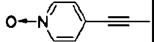 | Cl | CH | CH | CH | CCN | H |
| 96 | 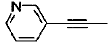 | 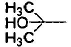 | CH | CH | CH | CCN | H |
| 97 | 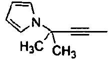 | Cl | CH | CH | CH | CCN | H |
| 98 | 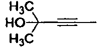 | Br | CH | CH | CH | CCl | H |
| 99 | 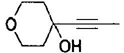 | Br | CH | CH | CH | CCl | H |
| 100 | Cl | CO₂i-Pr | CH | CH | CH | CCN | H |
| 101 | Cl | 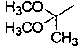 | CH | CH | CH | CF | H |
| 102 | 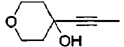 | Br | CH | CH | CH | CCN | H |
| 103 | 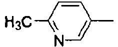 | Cl | CH | CH | CH | CCN | H |
| 104 | Br | 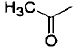 | CH | CH | CH | CCN | H |
| 105 | 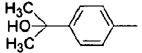 | Cl | CH | CH | CH | CCl | H |
| 106 | Br | 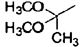 | CH | CH | CH | CCN | H |
| 107 | 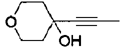 | Cl | CH | CH | CH | CCl | H |
| 108 | 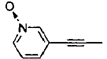 | Cl | CH | CH | CH | CCN | H |
| 109 | 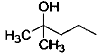 | Br | CH | CH | CH | CCN | H |
| 110 | 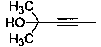 | Cl | CH | CH | CH | CCl | H |
| 111 | 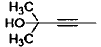 | 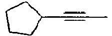 | CH | CH | CH | CCN | H |
| 112 | 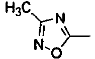 | Br | CH | CH | CH | CCN | H |
| 113 | 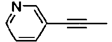 | 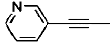 | CH | CH | CH | CCN | H |
| 114 | Et | 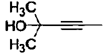 | CH | CH | CH | CCN | H |
| 115 | 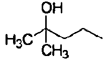 | 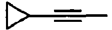 | CH | CH | CH | CCN | H |
| 116 | Br | 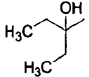 | CH | CH | CH | CCN | H |
| 117 | 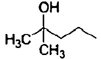 | Cl | CH | CH | CH | CCN | H |
| 118 | Br | CH3 | CH | CH | CH | CCN | H |
| 119 | 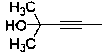 | CH3 | CH | CH | CH | CCN | H |
| 120 | 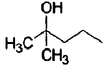 | CH3 | CH | CH | CH | CCN | H |
| 121 | 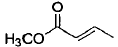 | Cl | CH | CH | CH | CCN | H |
| 122 | 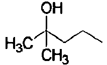 | H | CH | CH | CH | CCN | H |
| 123 | 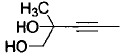 | Cl | CH | CH | CH | CCN | H |
ou un sel pharmaceutiquement acceptable de l'un quelconque des composés ci-dessus.

17. Composition pharmaceutique comprenant un composé selon l'une quelconque des revendications précédentes en combinaison avec un véhicule pharmaceutiquement acceptable.

18. Composé selon l'une quelconque des revendications 1-16, à utiliser dans le traitement d'une maladie ou d'une condition médiée par la prostaglandine E synthase-1 microsomale chez un patient humain ayant besoin d'un tel traitement.

19. Composé à utiliser selon la revendication 18, où la maladie ou condition est sélectionnée parmi le groupe consistant en: douleur aiguë ou chronique, ostéoarthrite, polyarthrite rhumatoïde, bursite, spondylite ankylosante et dysménorrhée primaire.
